# EUROPEAN PATENT APPLICATION

(11) **EP 0 816 375 A1**
(43) Date of publication of application: **07.01.1998**
(21) Application number: 96938222.5
(22) Date of filing: 15.11.1996
(51) Int. Cl.: C07H 17/02, C07H 17/00

(54) **GLYCOCONJUGATES OF OPIATED SUBSTANCES**

(30) Priority: 29.11.1995 ES 9502346
(71) Applicant: ROLABO SL, 50016 Zaragoza (ES)
(72) Inventor: COWIE, Diana, E-08160 Montmelo (ES); VALENCIA PARERA, Gregori, E-08160 Montmelo (ES)
(74) Representative: Cockbain, Julian, Dr.
(86) International application number: ES9600214
(87) International publication number: WO9721416

(57) **Abstract**

They are comprised of a series of derivatives of carbohydrates of a family of biologically active opiated agents, which present in their structure at least one residue of carbohydrate per opiate molecule, linked directly to a hydroxyl group of the opiate or through a C-glycoside bond formed between two functions situated at each of the two constitutive parts of the glycoconjugate. Said derivatives contain more than one carbohydrate residue and/or more than one opiate molecule per carbohydrate residue. The acid salts and the compounds of said opiated glycoconjugates are also included in the disclosed compounds of said invention.

## Description

In spite of at least five thousand years of long medicinal use of opium and more than a century experience on the therapeutic administration of one of its pure components (morphine) no significant progress has been made in avoiding the typical side effects of the opiate drugs such as tolerance, dependence, constipation and respiratory depression. A solution to these problems was suspected very close after the discovery of the endogenous opioids, the enkephalins in 1975. Owing to their endogenous nature it was thought that they may be free of side effects. However, very early studies showed that external administration of these peptides induced similar secondary effects as their opiate counterparts. In addition, as other peptides, the enkephalins have proved to be enzymatically labile and expensive to prepare and purify in large quantities. In summary, twenty years of intensive research on structure-activity relationship studies on these peptides have failed to provide neither a commercial analgesic drug of peptidic nature nor a solution to the side effects of opioids. As a consequence of this situation and although morphine and its synthetic analogues are far from the ideal prototype of an analgesic drug, they remain the most widely used phamaceuticals for the control of chronic pain. Hence, a typical dose of 10 mg of morphine delivered intravenously or intramuscularly, reduces awareness of pain, sedative effects, contraction of pupils and depression of cough reflex are always experienced by the patient. Other semisynthetic drugs, analogues of morphine which are currently used for the treatment of severe pain include hydromorphone, diamorphine (heroin) and oxymorphone. Similarly, codeine, dihydrocodeine and nalbuphine are also commonly employed for their milder analgesic properties. Codein is also an important antitussive and, thus, can be found as the main ingredient in a wide variety of cough mixtures. Other antitussive drugs are pholcodine, ethylmorphine, hydrocodeine and oxycodone. Along this family of morphine related structures , the opioid antagonist, naloxone, is used in the treatment of respiratory depression caused by opioid overdosis.

These semisynthetic opiates are commercially prepared by chemical modification of opium extracted morphine, codeine and thebaine. However, in spite that these chemical preparations are always difficult to achieve on a large scale and that precursors such as thebaine are often in limiting supply, factors such as low marked prices and extensive documented clinical use of morphine-related compounds still prevent other analgesic drugs to reach the pharmacopeia. With this in mind, feasible candidates to future analgesics are more likely to succed if they are based on current opiate structures. Towards the development of new pharmaceutical opiates showing better therapeutic profiles by modification of well known pain-related substances, strategies addressed at improving factors such as potency, half-life and blood-brain barrier passage are to be considered. In persuing this goal, other aspect of particular relevance that will be considered is the preparation of new opioid analogues less likely to have unwanted side effects such as respiratory depression and constipation.

Natural conjugation of medicinal drugs during metabolism often results in metabolites of reduced potency, therefore, conjugation was thought to terminate pharmacological activity of a compound. However, in recent years it has been disclosed that some of the glycoconjugate metabolites of morphine, namely morphine-6-glucuronide may be several times more active than the parent drug. The concentration of morphine-6-glucuronide in blood of patients taking morphine is two fold higher than morphine while the concentration of other metabolites such as morphine-3-glucuronide exceeds that of morphine approximately 20-fold. Interestingly enough, the 3-glucuronide is not analgesic but rather an antagonist of both morphine and the 6-glucuronide. In accordance, the analgesic effect of morphine is probably the result of the complex interaction and interconversion of the drug and this two main metabolites (J.G. Mulder, *Pharmacological effects of drug conjugates: is morphine 6-glucuronide an exception?* Trends in Pharmacol. Sci., 1992, 13, 302-304). The very opposite biological properties of these two natural analogues illustrate how crucial is the anchoring site of the glucuronide at confering analgesic properties.

In addition of being much more active than morphine when injected intrathecally, the 6-glucuronide of morphine also shows central analgesic action by peripheral administration. Apparently, the compound passes the blood-brain barrier (BBB) by diffusional transport more easily than anticipated from a polar structure of this kind. Recent conformational studies (P.A. Carrupt et al., *Morphine 6-glucuronide and morphine 3-glucuronide as molecular chamaleons with unexpected lipophilicity*., J. Med. Chem., 1991, 34, 1272-1275; P. Gaillart et al. *The conformation-dependent lipophilicity of morphine glucuronides as calculated from their molecular lipophilicity potential*, Bioorg. Med. Chem. Lett., 1994, 4, 5, 737-742) indicate that in aqueous media the structure of the glucuronides is extended to expose the polar groups for interaction with water molecules. However, a folded form that shields the polar groups is also possible which, in turn, may be responsible for the unexpected lipophilicity of these conjugates enabling their passage through biological membranes. The only experimental evidence available for the passage of the BBB by morphine glucuronides concerns physical diffusion transport but not involvement in mechanisms of active transport.

Applications of the above discussed properties of morphine glucuronides and of other opiate glucuronides are very few. Thus, the only patent exploiting the higher potency of the 6-glucuronide and its longer biological half-life for nasal delivery of opioid analgesics is Patent no. WO93/15737 (*Composition for nasal administration containing polar metabolites of opioid analgesics*). Moreover, before the knowledge of the analgesic properties of morphine glucuronides, only two patents, both based on the glucuronides of opioid antagonists had been filed. The main claim of Patent no. EP 0324212, 19.07.1989 (*Glucuronic acid derivatives of opioid antagonists*) is based on the properties of glucuronides of opiate antagonists such as nalmefene, naloxone and naltrexone for the treatment of constipation resulting from long exposure to opioids, while the second patent (Patent no. J 54105237, 1978, *Analgesic composition containing nalorphin-6-glucuronide*.) is centered on nalorphine.

The potential therapeutic applications of the naturally occurring morphine glucuronides has prompted the development of alternatives to early manufacturing procedures (i.e., H. Yoshimura et al. *Metabolism of drugs. LX. Synthesis of codeine and morphine* *glucuronides*., Chem. Pharm. Bull., 1968, 16, 11, 2114-2119; H. Yoshimura et al. *The synthesis of codeine and morphine D-glucuronides*., Tetrahedron Lett., 1968, 4, 483-486; B. Berrang et al. *Synthesis of morphine-3-glucuronide*, Synth. Commun. 1975, 5, 3, 231-236.). One of such methods is described in a recent patent granted for the manufacture of morphine glucuronides and a series of analogues (Patent. no. WO93/03051, 18.02.1993 *A process for making morphine 6-glucuronide or substituted morphine 6-glucuronide*.). A common feature of the analogues claimed on this patent is the presence of a glucuronate ester and/or substituted glucuronate ester on a morphine or substituted morphine molecule. This is to say that the core structure of these analogues is always the naturally occurring morphine-6-glucuronide structure where the nature and relative position of the bond linking the carbohydrate part and the opiate is identical to the natural parent compound.

Conjugation of pharmaceutical drugs other than glucuronidation, e.g. non-natural conjugation by chemical glycosidation with simple sugars, has not received much attention. Thus, only very recently such a strategy has been assayed on analgesic drugs. In particular, opioid' peptides rather than opiates have been examined. It has been found that sugar derivatives of two families of synthetically prepared enkephalin analogues are among the most potent opioid agonists already known. These glycopeptides induce profound and long lasting analgesia (R.E. Rodriguez et al., *New glycosylpeptides with high antinociceptive activity*., Neurosci. Lett., 1989, 101, 89-94 and R. Polt et al., *Glycopeptide enkephalin analogues produce analgesia in mice: Evidence for penetration of the blood-brain barrier*., Proc. Natl. Acad. Sci. USA, 1994, 91, 7114-7118). The rational idea for designing such molecular assemblies was to attach, to an opioid molecule which is not transported throughout the BBB, a substance like glucose which has its own active transporting system into the brain. In this way, it was expected that the glucose moiety on the peptide-sugar conjugate could be recognized by the glucose transporting system that, in turn, would bring the whole conjugate into the brain. In other words, the glucose moiety would function as a transport vector allowing the glycopeptide to be transported throughout the BBB up to the endogenous opioid receptors within the brain. It is worth noting that the first of the two publications demonstrates that opioid potency is a function of carbohydrate nature. Thus, the potency of a galactose analogue was approximately 1700 times higher than the corresponding one of glucose by intracerebroventricular (i.c.v.) administration. Although this fact may be partly explained in terms such as differences in transportability, diffusion and resistance to glycosidic enzymes, so far, the evidence available is still fragmentary and a full explanation awaits a complete structure-activity relationship study and further pharmacological and biochemical testing. Regretably, the main problem preventing a quick progress in this field is the intrinsic nature of glycopeptide molecules which present serious difficulties for synthesis and purification.

A second early example of peptide drugs that show improved properties when modified by simple sugar entities is presented in patent no. WO88/02756, 21.04.1988 (*Peptide derivatives*) which was later presented as a scientific publication (R. Albert et al. *SDZ CO 611: A highly potent glycated analog of somatostatin with improved oral activity*., Life Sci., 1993, 53, 517-525.). These documents describe a range of peptides such as somatostatin, calcitonin, LH-RH, oxytocin, vasopressin, insulin and growth hormone releasing factor modified by sugars throughout either Amadori or Heynes reactions.

In spite of the limited examples available of glycosidated drugs and the fact that the role of the carbohydrate part is far from being well stablished, there is an increasing number of glycoconjugate drugs that are now proving to be superior than the non-glycosidated counterparts for the treatment of various conditions. Examples of this growing evidence are the following. Dexamethasone-sugar conjugates have shown potential as prodrugs for colon specific delivery of these antiinflammatory substances (B. Haeberlin et al., *In vitro evaluation of Dexamethasone-β-D-glucuronide for colon-specific drug delivery*. Pharmaceutical Res., 1993, 10, 11, 1553-1562). Moreover, retinamide glycoconjugates have shown promise as more potent breast cancer chemopreventive agents than the non-glycosidated analogues (H. Abou-Issa et al. *In vivo use of N-(4-hydroxyphenyl retinamide)-O-glucuronide* as a breast cancer *chemopreventive agent*, Anticancer Res. 1993, 13, 1431-1436).

By the above considerations, it may be suspected that by choosing the right carbohydrate moiety and preparing a proper glycoconjugate drug it may be possible to modulate important features of a variety of therapeutic drugs. However, at the same time, serious drawbacks can also be foreseen when attempting to bring this theory into practice. These difficulties are well illustrated with the morphine glucuronides. As already discussed, two regioisomeric molecules, M3G and M6G, with the same glycosyl moiety on the same molecule but with different attachment site to the morphine core structure, with opposite biological functions are found in nature. In persuing the modulation of a particular biological feature by chemically bonding a sugar to an existing drug, this dramatic influence of just one factor, as shown in this example, enormously rises the complexity in finding the proper glycosyl moiety, a suitable chemical bond and a right bonding site that leads to a biologically active regioisomer. This is a straight consequence of the multifunctional character of sugar molecules which is often complicated by the various functional groups present on the structure of the drug. Moreover, an added difficulty is derived from the very few data available on the biological effects produced by glycosidation of pharmaceutical drugs. Owing to these facts, there is no garantee of success when extrapolation techniques of biological effects are used to choose among the different sugar-drug constructs that can be designed. This is even true when considering only one biological class of compounds and may be explained on the bases of every drug own structural features. An illustrative example of this rationale can also be found among analgesic compounds. It is well known that both morphine-related substances, the opiates, and enkephalin analogues, the opioid peptides, induce similar biological effects. This is so in spite of their very different chemical structures. In the case of the former, an alkaloid core structure, multifunctional rigid, polar and smaller molecule has nothing in common with the latter, a short peptide backbone, bifunctional, flexible, switterionic and larger molecule. In accordance, is at high risk of failure that extrapolation of the fragmentary biological data on glycosidation of opioid peptides, discussed above, can be considered of relevance when persuing, for instance, a similar approach for opiate molecules.

In spite of the above described uncertainties and difficulties we have been able to demonstrate that certain sugar derivatives (glycoconjugates) of a range of opiates, including morphine, may function as prodrugs. Thus, by making the right choice of sugar moiety, BBB passage may be facilitated, the analgesic potency increased and the therapeutic effect boosted. This, in turn, may contribute to lower the therapeutic doses of commonly used analgesic opioids and, eventually, reduce their insiduous side effects. In other words, by the present invention, a new range of synthetic opiate glycoconjugates which in a way mimic the natural morphine glucuronides is proposed as a novel class of analgesic substances. These so-called opiate glycoconjugates show a number of advantatges over their non-conjugated counterparts. The presence of a saccharide part make this hibrid molecules less prone to enzymatic degradation and thus improve their therapeutic life. Blood-brain barrier passage is also facilitated since the presence of saccharide moieties on the opiate molecules may fool the glucose active transport systems and thus allow the leaking of the opiate across the membranes. These proposed glycosilated molecular entities show potency enhancement than may be also explained by sugar-sugar affinity interactions between opioid receptors alredy composed by glycoproteins and the saccharide structures on the opiate.

In accordance, this invention relates to sugar derivatives of biologically active opiates. The object of the present invention is to provide a range of sugar derivatives of a series of biologically active opiates, such derivatives have enhanced analgesic potency if compared to the non-sugar modified opiates and carry at least one sugar residue per opiate molecule which is attached by direct O-glycosidic bond or ether coupling to one hydroxyl group of the opiate or by means of a C-glycosidic bond between two other suitable functions on the two constituting moieties. The sugar derivatives may also include in their molecule a linear or branched aliphatic or aromatic residue or a sulfate(s) or fosfate(s) group(s).

The compounds described above are referred from now on to as compounds of the invention and are also named opiate glycoconjugates.

The term non-sugar modified opiate is defined as the structurally corresponding opiate with no sugar residue attached onto its molecule. This is referred from now on to as unmodified opiate.

As described later the compounds of the invention show enhaced analgesic properties and we have found that such improvement is induced by the incorporation of the sugar residue or residues.

The term opiate as used here includes all non-peptide substances that bind specifically to opioid receptors including, e.g., morphinans, benzomorphans, methadones and phenylpiperidines. This class of compounds may also be termed as narcotic analgesics or morphine-like opiates.

By biologically active opiate is meant particular compounds having pharmacological and therapeutical activities in relation to analgesia, cough, dyspnea, constipation, anesthesia and diarrhea or which stimulate or inhibit such activity. These biologically active opiates include opiate substances isolated from nature or synthesized, also their derivatives or analogues are included in this group.

Under terms such as derivatives and analogues are included, in particular, both natural and synthetic opiates, wherein their basic opiate molecular pattern has been replaced by a different opiate molecular structure, e.g., morphinan by methadone motif, and/or wherein one or more functional groups have been replaced by one or several other isosteric groups. In a wider sense, the terms cover all modified derivatives of biologically active opiates which exhibit a qualitatively similar effect to that of the parent unmodified opiate.

The sugars or carbohydrates referred may be , e.g., any known mono- or oligosaccharide, especially a mono- di- or triose or a derivative thereof ,e.g., an amino and/or carboxylic acid and/or reduced and/or esterified and/or sulfated and/or phosphated derivative thereof.

The sugar moiety can be coupled ,e.g., to a hydroxyl group and/or tiol group and/or carbon atom of the opiate molecule.

The sugar may be coupled by one of its functional groups to the opiate either directly or indirectly by a linker or spacer unit, e.g, NHCO or NHCS. This coupling can be made by conventional means, especially as later described.

In a series of preferred compounds of the invention, the sugar residue is attached to an hydroxyl group of the opiate by an O-glycosidic bond. This group of compouds of the invention can be prepared by different methods of glycosidic bond formation, e.g., the Koenigs-Knorr reaction, Helferich, Hannessian or Schmidt procedures.

A series of compounds of the invention can be prepared by forming an ether bond between the opiate and the sugar molecules. Further series can be produced by C-glycosidic bond between these moieties.

The invention provides either oral or parenteral pharmacological preparations containing a compound of the invention especially those based on morphine.

The present invention provides, in particular, the following sugar derivatives of biologically active opiates of formulae:
a) 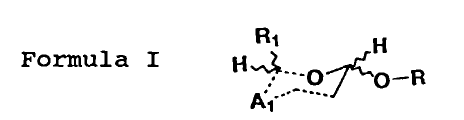 wherein 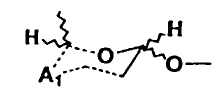 is a glycosyl residue being linked via glycosidic bond to an hydroxyl group of a biologically active opiate, R₁ is hydrogen, hydroxyl group, a saturated or insaturated or aromatic linear or branched or cyclic alcohol and/or acid residue with 1 to 18 C-atoms, a sulfate(s) or phosphate(s) residue(s) and R is the residue of a biologically active opiate of formula R-OH, wherein the OH group is either an alcohol or a phenol function.
b) 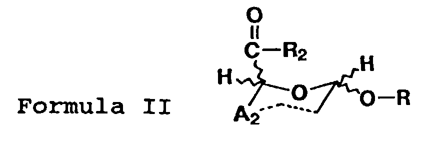 wherein 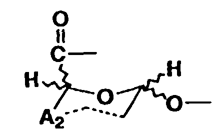 is a residue of an uronic acid, R₂ is hydroxyl, amino, a saturated or insaturated or aromatic linear or branched or cyclic amine or alcohol residue with 1 to 18 C-atoms and R is the residue of a biologically active opiate of formula R-OH, wherein the OH group is either an alcohol or a phenol function.
c)

   A₃-O-R Formula III

   wherein A₃-O- is a glycosyl residue being linked via ether bond to an hydroxyl group of a biologically active opiate and R is the residue of a biologically active opiate of formula R-OH, wherein the OH group is either an alcohol or a phenol function. d) 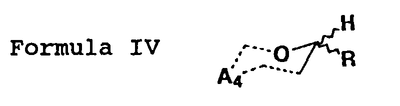 wherein 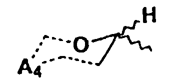 is a deoxy glycidyl residue being coupled via C-C single bond to an atom carbon of a biologically active opiate and R is the residue of a biologically active opiate.
e) 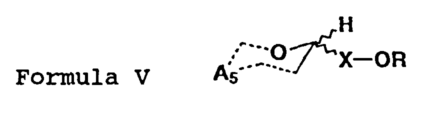 wherein 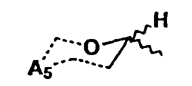 is a sugar residue, R denotes the residue of a biologically active opiate of formula R-OH and X is a linker group, i.e., NHCO or NHCS coupling the opiate to the sugar residue.

The acid salts and complexes of these opiate glycoconjugates are also considered among the compounds of the invention.

The above mentioned sugar residues may be mono-, di- or oligosaccharides. These sugars may contain heptoses, hexoses and/or pentoses, which, in turn, may be present in the form of pyranoses or furanoses.

In the given formulae I to V only one sugar moiety per opiate is given. However, the invention also covers sugar derivatives of opiates having more than one free hydroxyl group on the opiate molecule and therefore these structures may contain, e.g., 2 or 3 sugar residues per opiate unit. In accordance, the invention also provides all biologically active opiate derivatives which have more than one sugar residue coupled as above defined. These opiate glycoconjugates preferably contain 1 to 3 monosaccharide units which may also be joined in the active molecule as a disaccharide or trisaccharide.

Among other possibilities on the ratio of opiate per sugar molecules a particular one which involves a single sugar molecule covalently linked to more than one opiate molecule is also considered for the purpouses of the present invention.

In all the given formulae, the sinuous line ( ) means that the sugar units may be either in the form of the alfa or beta anomer.

In formula I the radical depicted as A₁ is preferably:
a) A residue of the formula VI.a 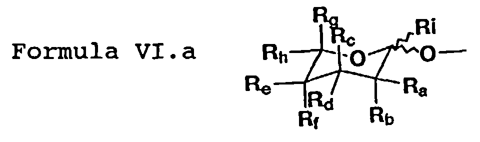 wherein one of the radicals Rₐ and R_{b} is hydrogen and the other is R₁ or NH₂ or NHOCOCH₃, one of the radicals R_{c} and R_{d} is hydrogen and the other is R₁ or O-glycosyl, being the glycosyl radical derived from a reducing mono-, di- or oligossacharide, one of the radicals, Rₑ and R_{f} is hydrogen and the other is R₁ or O-glycosyl, being the glycosyl radical derived from a reducing mono-, di- or oligossacharide, one of the radicals R_{g} and Rₕ is hydrogen and the other is CH₂-R₁ or O-glycosyl, being the glycosyl radical derived from a reducing mono-, di- or oligossacharide, the radial Rᵢ is hydrogen or CH₂OH, and the radical R₁ as described above, e.g., wherein radicals Rₐ to Rₕ are selected in such a manner that the residues of Formula VI.a correspond to a radical which is amenable by a glycosidic bond reaction formation from a natural or synthetically accesible mono-, di- or oligossacharide derivative.
   The following residues may be mentioned as examples of sugar residues of formula VI.a, glucosyl, galactosyl, mannosyl, fucosyl, xylosyl, rhamnosyl, glucosaminyl, N-acetyl-glucosaminyl, octylglucopyranosyl, ciclohexylglucopyranosyl, benzylglucopyranosyl, glucosyl sulfate, glucosyl phosphate, glucosaminyl sulfate, N-acetyl-glucosaminyl sulfate, lactosyl, gentiobiosyl, chitobiosyl, N-acetyl-lactosaminyl, cellobiosyl, maltosyl, melibiosyl and L-sorbosyl.
b) A residue of formula VI.b 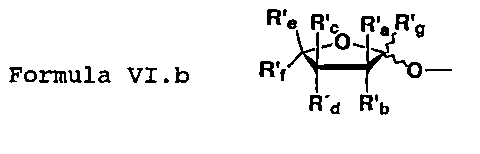 weherein one of the radicals R'ₐ and R'_{b} is hydrogen and the other is R₁ or O-glycosyl, being the glycosyl radical derived from a reducing mono-, di- or oligosaccharide, one of the radicals R'_{c} and R'_{d} is hydrogen and the other is R₁ or O-glycosyl, being the glycosyl radical derived from a reducing mono-, di- or oligosaccharide, one of the radicals R'ₑ and R'_{f} is hydrogen and the other is CH₂ or CHOH-CH₂OR₁ or O-glycosyl, being the glycosyl radical derived from a reducing mono-, di- or oligosaccharide, the radical R'_{g} is hydrogen or CH₂OH and the radical R₁ as described above, e.g., wherein radicals R'ₐ to R'_{g} are selected in such a manner that the residues of formula VI.b correspond to a radical which is accesible by a glycosidic bond reaction formation from a natural or synthetically produced mono-, di- or oligosaccharide derivative.

The following residues may be mentioned of sugar residues of formula VI.b, D-ribosyl, D-arabinosyl, D- xylosyl, D-lyxosyl, 2-deoxy-D-ribosyl, altosyl, idosyl, allosyl, D-fructosyl, D-sicosyl, D-sorbosyl, D-tagatosyl, D-xylulosyl, D-ribulosyl, D-glucofuranosyl, D-galactofuranosyl, octylribofuranosyl, cyclohexylxylofuranosyl, benzylarabinofuranosyl and D-fructosyl fosfate.

In formula II the structure referred as A₂ is preferably a residue of the formula VII. 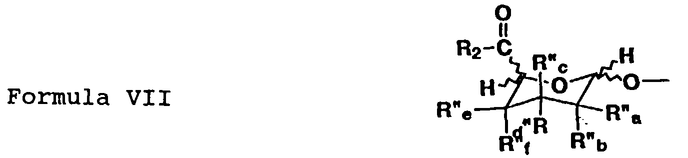 wherein one of the radicals R''ₐ and R''_{b} is hydrogen and the other is hydrogen, hydroxyl, amino or acetylamino group, one of the radicals R''_{c} and R''_{d} is hydrogen and the other is hydrogen or O-glycosyl, being the glycosyl radical derived from a reducing mono-, di- or oligossaccharide, one of the radicals R''ₑ and R''_{f} is hydrogen and the other is hydrogen or O-glycosyl, being the glycosyl radical derived from a reducing mono-, di- or oligossaccharide and the radical R₂ as defined above. Wherein, radicals R''ₐ to R''_{f} are selected in such a way that the residues of formula VII correspond to a radical which may be prepared by a glycosidic bond synthesis from a natural or synthetically accesible mono-, di or oligosaccharide derivative.

Residues of formula VII may be attained, for example, by glycosidic coupling from sugar derivatives of the uronic acid series such as glucuronic acid, galacturonic acid, glucuronamide, glucosaminuronic acid, octylgalacturonamide, cyclohexylgalacturonate, decylglucoronate and benzylglucoronamide. Furthermore, other complex sugar acids such as muramic acid, neuraminic acid, acetyl muramic, acetyl neuraminic and sialic acid are also included as examples of compounds meant by sugar acid derivatives others than of formula VII which are also claimed as residues forming the compounds of the invention.

In formula III the radical indicated as A₃ have the definitions already given for A₁ and A₂ by formulae VI.a, VI.b and VII.

For compounds of formula IV the preferred ones are those whose radical A₄ is defined by formulae VI.a, VI.b and VII, especially when a monosaccharide derivative is considered.

Preferred compounds of formula V are the ones presenting an A₅ residue as described by formulae VI.a, VI.b and VII, in particular, X denotes radicals such as NHCO and NHCS.

In order to prepare the desired glycoconjugates, all those described sugar residues have to be covalently coupled to an appropiate R radical of a biologically active opiate. Such opiates include all natural and synthetic opiates, derivatives and analogues having activity on pain, cough, dyspnea, diarrhea and anesthesia. They action may be both stimulating and inhibiting. The following may be mentioned as examples of such opiates: morphine, codeine, oxymorphone, naloxone, nalbuphine, buprenorphine, pholcodine, meperidine, loperamide, sufentanil, methadone, propoxyphene, metazocine, phenazocine and pentazocine.

Preferred glucoconjugates are those of formulae I and II.

Although in the already given structures sugar radicals are either depicted as pyranoses or furanoses, the invention includes any of these structures provided that they exist for the relevant sugar.

The present invention includes the processes for the preparation of compounds of the above formulae. They may be produced by methods which are well known for this type of substances. A general example of preparation of the compounds of the invention may be as follows:
a) At least one protecting group on the molecule of the opiate glyconjugate is cleaved or,
b) A conveniently modified and/or protected sugar residue and a suitably derivatized opiate unit are linked together by glycosidic or ether or C-C bond, and stage a) of the process is optionally performed.
c) At least one optionally protected residue is introduced into a protected or unprotected opiate glycoconjugate and stage a) of the process is optionally effected, or
d) A functional group of a protected or unprotected opiate glycoconjugate is converted into another functional group or removed and a further residue is introduced, so that an unprotected or protected opiate glycoconjugate is obtained, and in the latter case, stage a) is followed.

These reactions may be conducted by conventional means analogously to the processes described in the following general examples. In particular, stages a) and b) may be carried out according to the synthesis of the invention later described. These procedures always require the use of suitable protecting groups for reactive functions in both the sugar and the opiate moiety to prevent them from participating in the reaction.

The compounds of formula I may be prepared by reacting a protected opiate (acyl acceptor) having a free hydroxyl group with an activated reducing mono-, di- or oligosaccharide derivative (acyl donor) in the presence of a promoter (conjugation reaction) and subsequently removing the protecting group(s).

When a modification on the sugar molecule is to be introduced a separate reaction step is required. This additional step can be effected before or after the conjugation reaction and may involve ether bond formation or phosphate of sulfate group bonding.

The conjugation reaction may take place in a conventional way for a glycosidic bond formation. The promoter used may be, e.g., a Ag⁺ or Hg⁺⁺ compound or a Lewis acid such as BF₃OEt₂. A tertiary organic amine may be added. The reaction may be preferably carried out in solvents such as dichloromethane and acetonitrile at temperatures on the range of subzero to room temperature.

The compounds of formula II may be prepared by reaction between a protected opiate having a free hydroxyl group and a uronic acid derivative. The conjugation reaction and additional steps for sugar moiety modification may be performed as for the case of compounds of formula I. In this case, sugar moiety modification may involve other reaction procedures such as ester or amide bond formation that may be carried out by conventional well stablished procedures such as acid halides, carbodiimides, phosphonium or uronium salts, active esters and symmetrical anhydrides.

The compounds of formula III can be produced by reacting a protected opiate having a free hydroxyl group with a free hydroxyl group of a reducing or not-reducing mono-, di-, or oligosaccharide by forming an ether bond and then removing the protecting group(s). This procedure may be a conventional ether bond reaction which may be effected in a known way. Thus, the opiate is reacted with triflate anhydride in the presence of a tertiary base at low temperature and subsequently the sugar derivative is slowly added. After work-up the protecting groups are removed.

The compounds of formula IV can be produced by reacting a nucleophilic derivative of an opiate with an electrophilic sugar derivative to yield a C-C bond between the opiate and the anomeric position of the sugar moiety and then removing the protecting group(s). Examples of electrophilic species that can be used are: halides, imidates, lactones, glycals, thioglycosides and O-glycosides such as p-nitrobenzoates. As nucleophilic derivatives silyl enol ethers, alquenes, allylsylanes, allylstannanes and organometallic compounds such as Grignard reagents, organolitics, cuprates and aluminates can be employed. These procedures involve several step reactions that can be conducted in known ways.

Compounds of formula V may be produced for example by coupling the protected corresponding glycosyl isocyanates or glycosylthioisocianates to a R-OH protected opiate and subsequently removing the protecting groups. This reaction may be carried out by conventional means for the synthesis of urethane or thiourethane derivatives.

The preparation of the raw materials for these reactions is not described here, however, it is a known procedure or may be achieved by conventional means, e.g., using known methods described in the literature for analogous compounds or described here by the syntheses of the invention.

One preferred class of compounds of the invention comprises the sugar derivatives of opiates structurally related to morphine. The term opiates structurally related to morphine includes opiates of morphinan structure their analogues or derivatives. Especially preferred are the sugar derivatives of compounds of formula VIII. 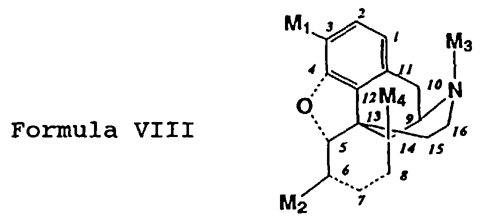 wherein, M₁ is hydroxyl, methoxy, ethoxy, acetoxy or 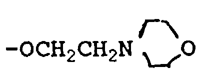 group; M₂ is hydrogen, hydroxyl, oxy, acetoxy, methoxy and methene group; M₃ is methyl, methylen cyclopropyl, methylen cyclobutyl and allyl group; M₄ hydrogen or hydroxyl; the bond between carbon atoms in positions 6-7 and 7-8 can be either single or double; the ether function between position 4 and 5 can be either present or not present on the molecule. Compounds of formula VIII can either exist in the form of levo or dextrorotatory isomers as well as salts and complexes.

Among the opiate derivatives of formula VIII, the following definitions or combinations are preferred.

If M₁ and M₂ are hydroxyl, M₃ methyl, M₄ hydrogen and a double bond between positions 7-8 and ether function between 4-5 exist, morphine is then defined.

If M₁ is hydroxyl, M₂ is oxy, M₃ allyl, M₄ hydroxyl and a single bond between positions 7-8 and ether function between 4-5 exist, naloxone is then defined.

Accordingly definitions for codeine, heroin, hydromorphone, hydrocodone, oxymorphone, oxycodone, levorphanol, dextromethorphan, nalorphine, naltrexone, levallorphan, thebacone, ethyl morphine, dihydrocodeine, nalmefene, nalbuphine, butorphanol and pholcodine can be deduced. Other analogues such as metopon and buprenorphine that can not be deduced by the general formula VIII are also preferred morphinan compounds.

Especially preferred sugar derivatives of morphinan structure are those which have a sugar residue on either positions C-3 and C-6 of the morphinan backbone, e.g., compounds of formula:

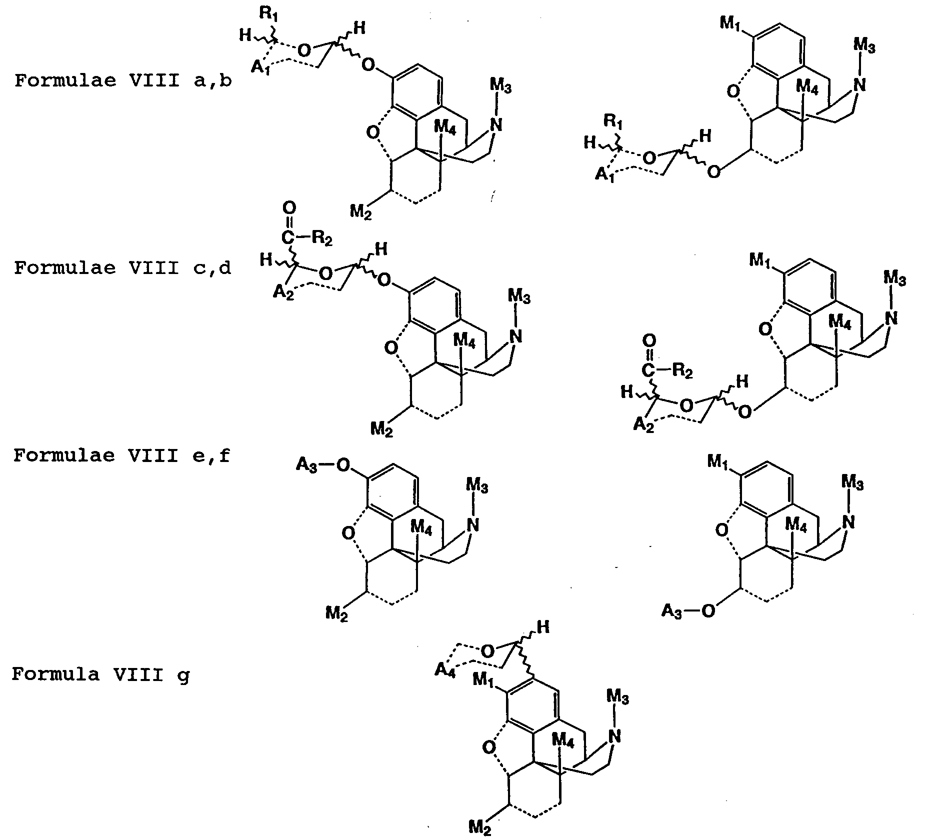

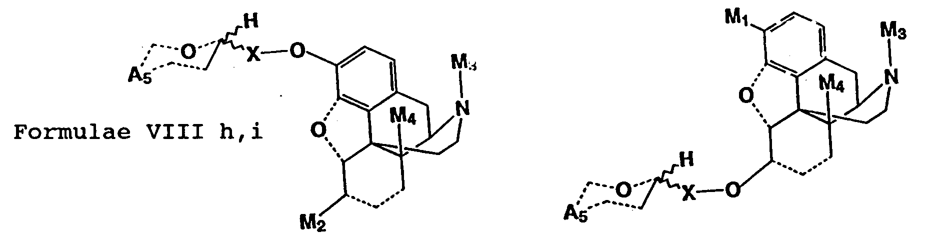

Especially preferred compounds are those of formulae VIII a,b and VIII c,d for morphine, codeine and naloxone.

A further preferred class of compounds of the invention comprises the sugar derivatives of opiates of the meperidine (pethidine) series. The term opiates of meperidine series refers to the phenylpiperidine type of opiates and derivatives and analogues. Derivatives and analogues of opiates of the meperidine series include piperidine type of structures wherein radicals have been modified to introduce suitable functions such as hydroxyl groups that may allow further coupling reactions with sugar units.

Especially preferred are the sugar derivatives of opiates of the meperidine series of the following formula IX. 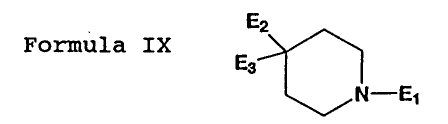 wherein E₁ is methyl, phenylethyl, p-phenylethylamine,
-(CH₂)₃-NH-C₆H₅,
-(CH₂)₂-C(C₆H₅)₂-CN, -(CH₂)₂-C(C₆H₅)₂-CO-N(CH₃)₂, 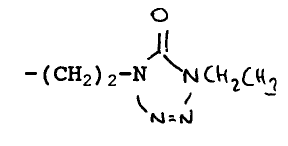 and 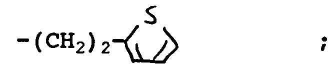 E₂ is hydrogen, phenyl, m-hydroxyphenyl, p-chlorophenyl, methylenemetoxide
and piperidinyl; E₃ is hydroxyl, ethylcarbonyl, carboxamide, ethyloxycarbonyl, ethylcarboxyl and -N(C₆H₅)-CO-CH₂-CH₃.

Combinations and definitions preferred for opiates of formula IX are the following.

If E₁ is methyl, E₂ phenyl, and E₃ ethoxycarbonyl, meperidine is then defined.

If E₁ is -(CH₂)₂-C(C₆H₅)₂-CN, E₂ phenyl and E₃ ethoxycarbonyl, diphenoxylate is then defined.

In accordance definitions for loperamide, fentanyl, sufentanil, alfentanil, cetobemidon, piritramide, anileridine, piminodine can be deduced. Other analogues such as alphaprodine, fenpipramide, ethoheptazine, tilidin and nefopam that can not be deduced by the general formula IX are also preferred opiate compounds of the meperidine series. The glycosilated opiates of the meperidine series, including derivatives and analogues, are especially those which are glycosilated on one hydroxyl group already present or introduced for binding purposes on radicals E₂ and E₃ of formula IX, e.g., compounds of following formulae. 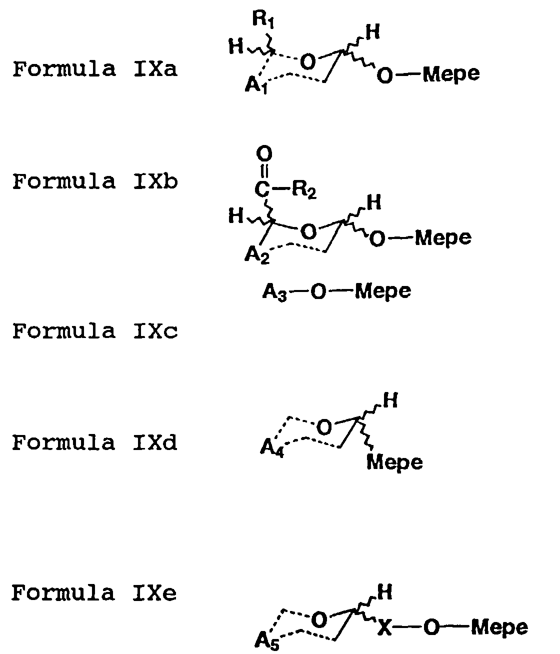 **Mepe** denotes the residue of meperidine or of a derivative or analogue of meperidine which is bonded to the sugar residue via an hydroxyl group alredy existing such as in the case of loperamide and cetobemidon or introduced for coupling purposes into one of the radicals E₂ and/or E₃ of formula IX.

Especially preferred compounds are those of formula IXa and IXb for meperidine, loperamide and cetobemidon.

Other series of compounds of the invention comprises the sugar derivatives of opiates of the methadone type. The term opiates of the methadone type refers to opiate substances with structure clossely related to methadone as well as derivatives and analogues. Derivatives and analogues of methadone include structures wherein radicals have been modified with functions such as hydroxyl groups to allow conjugation with sugar compounds.

Especially preferred are the sugar derivatives of opiates of the methadone type of the following formula X. 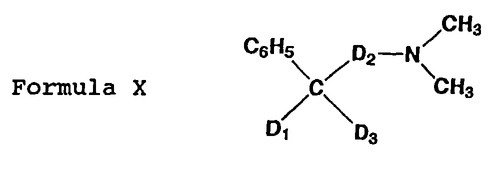 wherein D₁ is phenyl or benzyl; D₂ is -CH₂-CH(CH₃)-, -CH(CH₃)-CH₂-and -(CH₂)₂-; D₃ is ethylcarbonyl, ethylcarboxyl and -CH(OCOCH₃)-CH₂-CH₃.

Definitions and combinations preferred for opiates of formula X are the following.

If D₁ is phenyl, D₂ -CH₂-CH(CH₃)- and D₃ ethylcarbonyl, methadone is defined.

If D₁ is benzyl, D₂ -CH(CH₃)-CH₂- and D₃ ethylcarboxyl, propoxyphene is defined.

Analogously, definitions for normethadone and methadyl acetate can be deduced. Other analogues such as dextromoramide that can not be deduced from the general formula X are also preferred opiates of the methadone type of compounds.

The sugar conjugates of opiates of the methadone type, including derivatives and analogues, are especially those which are glycosilated on one hydroxyl group introduced for coupling purposes on radicals D₁ and D₃ of formula X, e.g., compounds of following formulae.

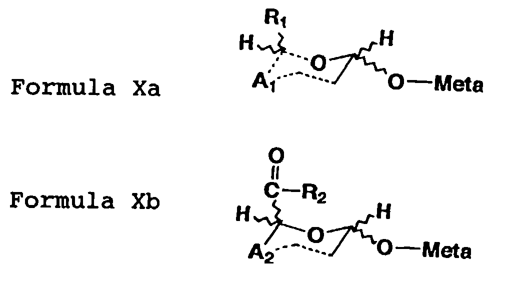

A₃-O-Meta Formula Xc

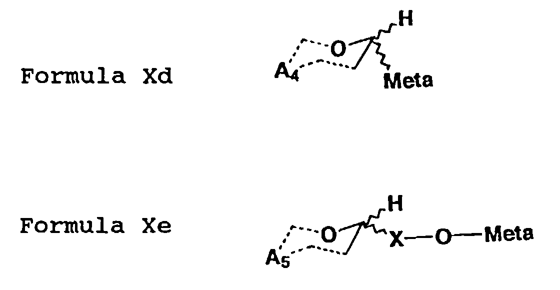

**Meta** refers to the residue of methadone or to a derivative or analogue of methadone which is coupled to a sugar residue via an hydroxyl group introduced for coupling purposes into one of the radicals D₁ and/or D₃ of formula X.

Especially preferred compounds are those of formula Xa and Xb for methadone.

A final series of preferred compounds of the invention comprises the sugar derivatives of opiates of the benzomorphane group. The term opiates of the benzomorphane group refers to opiate substances with structures that respond to benzomorphane motif as well as derivatives and analogues.

Especially preferred are the sugar derivatives of opiates of the benzomorphane group of the following formula XI. 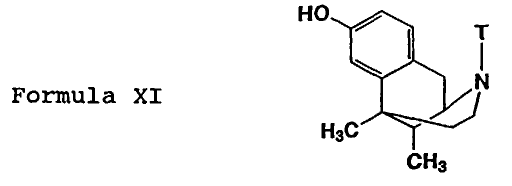 wherein T is methyl, phenylethyl, methylcyclopropyl and -CH₂-CH=C(CH₃)₂

Definitions preferred for opiates of formula XI that can be deduced from such a formula are the following.

If T is methyl, phenylethyl, methylcyclopropyl and -CH₂-CH=C(CH₃)₂, methazocine, phenazocine, ciclazocine and pentazocine are respectively defined.

Other analogues such as dezocine and meptazinol that can not be deduced from the general formula XI are also preferred opiates of the benzomorphane group of compounds.

The sugar conjugates of opiates of the benzomorphane group, including derivatives and analogues, are especially those which are glycosilated on the phenol function of formula XI, e.g., compounds of the following formulae:

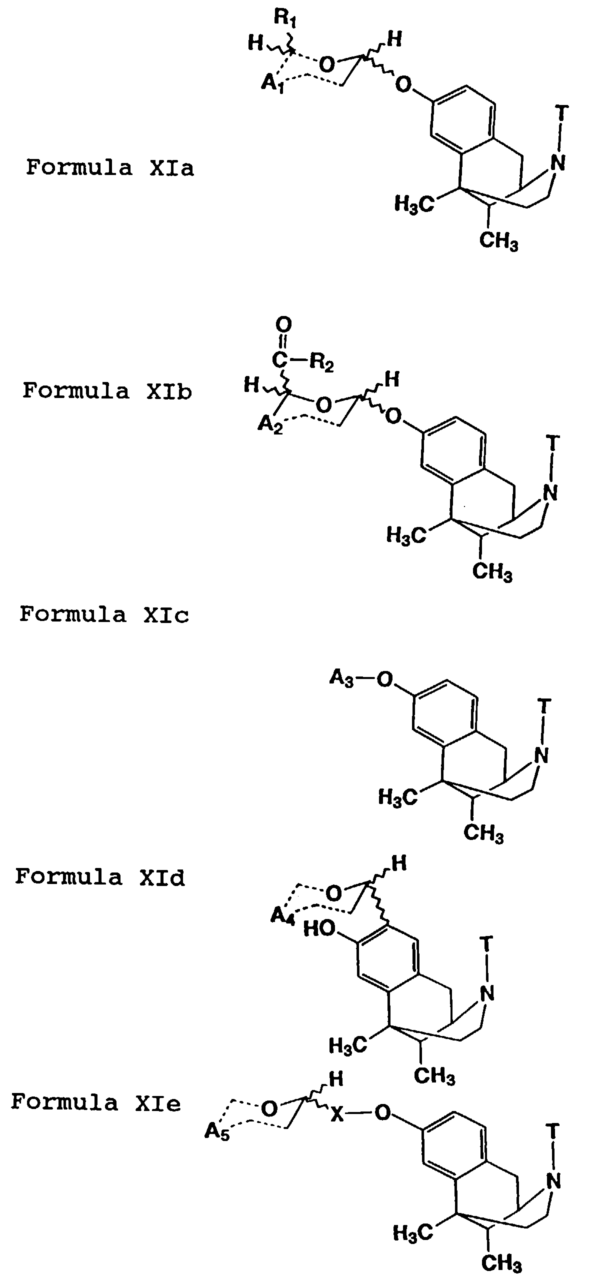

Especially preferred compounds are those of formula XIa and XIb for methazocine, pentazocine and cyclazocine.

### EXAMPLES

In the example section of this patent, temperatures are given in degrees Celsius. Abbreviations used are as follows:
HOBt = N-hydroxybenzotriazole.
AcOH = acetic acid.
DMF = dimethylformamide.
TBTU = 2-(1H-benzotriazole-1-yl)-1,1,3,3-tetramethyluronium tetrafluoroborate.
HBTU = 2-(1H-benzotriazole-1-yl)-1,1,3,3-tetramethyluronium hexafluorophosphate.
MeOH = methanol.
DIC = dicyclohexylcarbodiimide.
Triflate = trifluoromethane sulfonate.
ESI-MS = electrospray ionization mass spectrometry.
BOP = Benzotriazole-1-yl-oxy-tris-(dimethylamino)-phosponium hexafluorophosphate.
TEMPO = 2,2,6,6 Tetramethyl-1-piperidinyloxy, free radical.
¹H NMR = Proton nuclear magnetic resonance spectroscopy.
¹³C NMR = Carbon nuclear magnetic resonance spectroscopy.
M3G = Morphine 3-glucuronide.
M6G = Morphine 6-glucuronide.

### Example 1.

### 3-Morphinyl n-octyl-(β-D-glucopyranosyl)-uronamide acetate.

To a solution of 0.50 g of 3-morphinyl-β-D-glucopyranosyl uronic acid acetate, 0.14 g of n-octylamine and 0.15 g of HOBt in 20 ml of DMF, a second solution of 0.35 g of TBTU in 10 ml of DMF are added. The reaction is kept overnight under stirring at room temperature. After solvent evaporation, the crude product is purified by column chromatography on silica gel using as solvent a mixture of ethyl acetate/water/methanol (9/4/5). The title compound is isolated as a white lyophilizate from an aqueous acetic acid solution.
ESI-MS: 595.5 [M+Na]⁺, ¹H and ¹³C NMR data in accordance with the expected structure, [&]²²_{D} = -124.5 (c=1, MeOH).

The starting products may be produced as follows.

### a) 3-Morphinyl (β-D-glucopyranosyl)-uronic acid acetate.

To a solution of 4.00 g of morphine free base and 0.50 g of lithium hydroxide monohydrate in 20 ml of methanol are added 5.50 g of methyl-(tri-O-acetyl-&-D-glucopyranosyl bromide)-uronate. The mixture is stirred at room temperature for 30 min. A solution of 1.40 g of lithium hydroxide monohydrate in 20 ml of water is then added. After further 30 min, the reaction is brought to pH:8 with acetic acid and the unreacted morphine is filtered off. The filtrate is evaporated and the crude residue is precipitated with a mixture of methanol/acetone. The solid fraction is further purified on a silica gel column chromatography using a solvent mixture of ethyl acetate/water/methanol (2/3/1). After lyophilization from an aqueous acetic acid solution the title compound is isolated as a white powder.
ESI-MS: 484.2 [M+Na]⁺ and 462.3 [M+H]⁺, [&]²²_{D} = -110.5 (c=0.55, H₂O), ¹H and ¹³C NMR data in accordance with the expected structure. b) Methyl-(tri-O-acetyl-&-D-glucopyranosyl bromide)-uronate.

A solution of 10.00 g of methyl-(tetra-O-acetyl-β-D-glucopyranosyl)-uronate in 40 ml of 30% hydrobromic acid in acetic acid is prepared and kept overnight at 0ºC. The solvent is evaporated and the residue dissolved in HCCl₃. The solution is extracted with cold aqueous bicarbonate and water. After drying, the solvent is evaporated and the residue crystallized from ethanol. The title compound is obtained as a white crystalline solid. [&]²²_{D} = 195.9 (c=1, HCCl₃)

### c) Methyl (tetra-O-acetyl-ß-D-glucopyranosyl)-uronate.

To a solution of 30 mg of sodium hydroxide in 100 ml of MeOH 10.00 g of D-glucurono-6,3-lactone are added. The mixture is stirred at room temperature for 1 hr and the solvent evaporated. After cooling to 0ºC the residue is dissolved in a mixture of 25 ml of pyridine and 40 ml of acetic anhydride and left overnight under stirring. Solvent evaporation and crystallization from chloroform/petroleum ether yields the title compound as a white crystalline solid. M.P. 178-180ºC, [&]²²_{D} = 7.5 (c=1, HCCl₃)

### Example 2.

### 3-Morphinyl n-octyl (β-D-galactopyranosyl) uronamide acetate.

The corresponding galacturonic acid analogue is prepared by a similar procedure as described in example 1.

### Example 3.

### 6-Morphinyl n-octyl(β-D-glucopyranosyl)-uronamide acetate.

To a solution of 1.72 g of 6-(3-O-acetyl)-morphinyl n-octyl-(β-D-tri-O-acetyl-glucopyranosyl)-uronamide in 25 ml of absolute MeOH, 10 ml of 1% sodium methoxide in methanol are added. After overnight at room temperature, the reaction is neutralized with acetic acid and the solvent removed. The crude residue is purified by chromatography on a silica gel column eluted with an ethyl acetate/water/methanol (9/4/5) mixture. Lyophilization from an aqueous acetic acid solution yields the title compound as a white solid.
ESI-MS: 595.5 [M+Na]⁺ and ¹H and ¹³C NMR data in good agreement with the proposed structure.

The starting products may be produced as follows.

### a) 6-(3-O-Acetyl)-morphinyl n-octyl-(β-D-tri-O-acetyl-glucopyranosyl)-uronamide.

In a moisture free system, a solution of 2.20 g of n-octyl-(tetra-O-acetyl-β-D-glucopyranosyl)-uronamide and 1.50 g of 3-O-acetyl-morphine in 50 ml of dry dichloromethane and 3Å molecular sieves is prepared. To this solution 3.3 ml of boron trifluoride ethyl eterate complex are dropwise added. The reaction mixture is succesively filtered, quenched over ice and extracted with chloroform. After drying over anhydrous sodium sulfate, the solvent is evaporated under reduced pressure and the residue is further purified. Column chromatography on silica gel eluted with ethyl acetate/petroleum ether (9/1) mixture yields a white solid identified as the title compound.
ESI-MS: 763.8 [M+Na]⁺ and 741.6 [M+H]⁺, ¹H and ¹³C NMR data in good agreement with the proposed structure.

### b) n-Octyl-(tetra-O-acetyl-ß-D-glucopyranosyl)-uronamide.

A solution of 4.00 g of tetra-O-acetyl-D-glucopyranosyl-uronic acid, 1.40 g of n-octyl amine and 1.50 g of HOBt in 30 ml of dichloromethane is prepared. A second solution of 1.40 g of DIC in 10 ml of dichloromethane is added and the mixture left overnight under stirring at room temperature. The urea is filtered and discarded and the filtrate evaporated under reduced pressure. The waxy residue is crystallized from ethyl acetate-petroleum ether yielding the title compound.
M.P. 133-134ºC, ESI-MS: 496.6 [M+Na]⁺, ¹H data in accordance with the proposed structure.

### c) Tetra-O-acetyl-D-glucopyranosyl-uronic acid.

A solution of 47.00 g of D-glucurono-6,3-lactone and 25.00 g of sodium hydrogen carbonate in 175 ml of water is stirred overnight at room temperature. The solvent is evaporated under reduced pressure and the residue triturated with ethanol. After drying, 63.00 g of this yellow solid residue are dissolved in a stirred solution of 65.00 g of p-toluensulphonic acid monohydrate in 230 ml of acetic anhydride. The reaction temperature is mantained at 0ºC for 1 hr. After this time the mixture is diluted with 2 volumes of ethyl ether and whased with a solution of 7.00 g of sodium acetate in 200 ml of water. The organic layer is dried over anhydrous sodium sulfate, filtered and the solvent removed. The yellow residue is further crystallized from acetone/water yielding the title compound.
M.P. 110-112ºC, ESI-MS: 385.0 [M+Na]⁺, ¹H data in accordance with the structure of title compound.

### d) 3-O-Acetyl-morphine.

To a 800 ml solution of 10% sodium hydrogencarbonate, 8.00 g of morphine free base are added. Addition of 40 ml acetic anhydride was achieved by vigoruos stirring and occasional ethyl ether dropping to prevent foaming. After 20 min at room temperature, ice is added and the mixture extracted with chloroform. The organic layers are dried over anhydrous sodium sulfate and filtered. Removal of the solvent in vacuo yields the title compound as a slightly yellow foaming solid.

The example compound may be achieved throughout a second reaction pathway by means of the following intermediates.

### a.1) 6-(3-O-Acetyl)-morphinyl n-octyl-(β-D-tri-O-acetylglucopyranosyl)-uronamide.

Under argon atmosphere, to a -40ºC cooled solution of 1.10 g of 3-O-acetyl-morphine and 0.85 g of silver triflate in 20 ml of dry dichloromethane in the presence of 3Å molecular sieves, a second solution of 1.60 g of n-octyl-(tri-O-acetyl-&-D-glucopyranosyl bromide)-uronamide in 10 ml of dry dichloromethane are added dropwise. The reaction is kept at -40ºC under stirring for 3 hr and 0.5 ml triethylamine are then added. Once the reaction reaches room temperature, the silver salts are filtrated off and the filtrate is evaporated in vacuo. The residue is purified on silica gel eluting with a chloroform/MeOH mixture (9.5/0.5) to yield the title compound.
ESI-MS: 763.4 [M+Na]⁺ and 741.7 [M+H]⁺, ¹H and ¹³C NMR data in good agreement with the proposed structure.

### b.1) n-Octyl-(tri-O-acetyl-&-D-glucopyranosylbromide)-uronamide.

A solution of 2.30 g of n-octyl-(tetra-O-acetyl-β-D-glucopyranosyl)-uronamide in 10 ml of 30% hydrobromic acid in acetic acid is prepared and kept overnight at 0ºC. The solvent is evaporated and the residue dissolved in HCCl₃. The solution is extracted with cold aqueous bicarbonate and water. After drying, the solvent is evaporated and the residue purified by a silica gel column eluted with ehtyl acetate/petroleum ether (5/5). The amorphous solid thus prepared corresponds to the structure of the title compound by ¹H NMR spectroscopy.

A third reaction pathway leading to the example compound may be carried out throughout the following intermediates.

### a.2) 6-(3-0-Acetyl)-morphinyl n-octyl-(β-D-tri-O-acetyl glucopyranosyl)-uronamide.

To a solution of 0.15 g of 6-(3-O-acetyl)-morphinyl (tri-O-acetyl-β-D-glucopyranosyl)-uronic acid and 0.03 g of n-octyl amine in 10 ml of DMF, a solution of 0.10 g of BOP in 5 ml of DMF are added. The reaction is stirred at room temperature for 1 hr. After this time, the mixture is diluted with HCCl₃ and extracted with water. The organic layer is dried over anhydrous sodium sulfate, filtered and the solvent removed. The residue is further purified on a silica gel column with a solvent mixture of ethyl acetate/petroleum ether (9/1) yielding the title compound as a white solid.
ESI-MS: 763.7 [M+Na]⁺ and 741.5 [M+H]⁺, ¹H and ¹³C NMR data in accordance with the proposed structure.

### b.2) 6-(3-O-Acetyl)-morphinyl (tri-O-acetyl-β-D-glucopyranosyl)-uronic acid,

Under argon atmosphere, to a solution of 0.20 g of 3-O-acetyl morphine, 0.22 g of tetra-O-acetyl-D-glucopyranosyl uronic acid in 5 ml of Cl₂CH₂ in the presence of 3A molecular sieves, 250 µl of BF₃.Et₂O complex are added. The reaction temperature is kept at 0ºC during the addition and is later left to rise to room temperature. After 24 hr the reaction is quenched on ice and extracted with HCCl₃. The organic layer is dried on anhydrous sodium sulfate, filtered and the solvent removed in vacuo. Chromatography on silica gel using a mixture of chloroform/methanol/acetic acid (4/6/1) as a eluent, yields the pure title compound as a white solid.
ESI-MS: 652.5 [M+Na]⁺ and 630.6 [M+H]⁺, ¹H and ¹³C NMR data in accordance with the proposed structure.

An alternative procedure may be as follows.

To a solution of 0.68 g of 3-O-acetyl-morphine and 0.53 g of silver triflate in 10 ml of dry dichloromethane in the presence of 3Å molecular sieves and under argon atmosphere, a solution of tri-O-acetyl-&-D-glucopyranosyl bromide uronic acid is slowly added. The reation is kept at -40ºC under stirring for 4 hr and 0.3 ml of triethylamine are added. After bringing the reaction at room temperature and filtering the silver bromide, the filtrate is evaporated. The resulting residue is purified by column chromatography as described above, yelding the title compound.
ESI-MS: 652.7 [M+Na]⁺ and 630.4 [M+H]⁺, ¹H and ¹³C NMR data in accordance with the proposed structure.

### c.2) Tri-O-acetyl-&-D-glucopyranosyl bromide uronic acid.

A solution of 2 g fo tetra-O-acetyl-D-glucopyranosyl uronic acid in 10 ml of 30% hydrobromic acid in acetic acid is prepared and kept overnight at 0ºC. The solvent is evaporated and the residue crystallized in diethyl ether/hexane. The white solid is identified as the title compound.

¹H and ¹³C NMR data in accordance with the proposed structure.

A fourth reaction pathway may also be followed in the preparation of the title compound. This route is indicated by the intermidiates described below.

### a.3) 6-Morphinyl n-octyl-(β-D-glucopyranosyl)-uronamide acetate.

To a solution of 0.50 g of 6-morphinyl-β-D-glucopyranosyl uronic acid acetate, 0.14 g of n-octylamine and 0.15 g of HOBt in 20 ml of DMF, a second solution of 0.35 g of TBTU in 10 ml of DMF are added. The reaction is kept overnight under stirring at room temperature. After solvent evaporation, the crude product is purified by column chromatography on silica gel using as solvent a mixture of ethyl acetate/water/methanol (9/4/5). The title compound is isolated as a white lyophilizate from an aqueous acetic acid solution.
ESI-MS: 595.6 [M+Na]⁺, ¹H and ¹³C NMR data in accordance with the expected structure.

### b.3) 6-Morphinyl β-D-glucopyranosyl uronic acid acetate.

A suspension of 1 g of 6-(3-acetyl)-morphinyl methyl-(tri-O-acetyl-β-D-glucopyranosyl)-uronate in 20 ml of absolute methanol, 8 ml of 1% sodium methoxide in methanol are added. After overnight at room temperature the mixture is concentrated to dryness and the residue dissolved in 10 ml of 0.40N barium hydroxide and kept for 1 hr at room temperature. After this time the pH is adjusted to 8.0 with 2N oxalic acid and the barium salts filtered off. The filtrate is evaporated to dryness and the residue purified by a silica gel column eluted with a solvent mixture of ethyl acetate/water/methanol (2/3/1). After lyophilization from an aqueous acetic acid solution the title compound is isolated as a white solid.
ESI-MS: 484.3 [M+Na]⁺ and 462.2 [M+H]⁺, [&]²²_{D} = -263.0 (c=0.50, H₂O), ¹H and ¹³C NMR data in accordance with the expected structure.

### c.3) 6-(3-Acetyl)-morphinyl methyl-(tri-O-acetyl-β-D-glucopyranosyl)-uronate.

In a moisture protected system, a suspension of 0.80 g of 3-O-acetyl-morphine in 80 ml of dry bencene and 2 g of dry AgCO₃ is prepared. To this mixture 1.5 g of methyl-(tri-O-acetyl-&-D-glucopyranosylbromide)-uronate are added in several portions during a 9 hr period while keeping the suspension stirred and heated to reflux. Over this time, bencene is gradually distilled off and the filtrate concentrated and extracted with ice-cool HCl 0.5%. The aqueous layer is adjusted to pH: 8.0 with sodium hydrogen carbonate and extracted with HCCl₃. Chloroform extracts are dried and evaporated to dryness yielding a residue which is purified by silica gel column eluted with a HCCl₃/MeOH mixture. The resulting white solid is identified as corresponding to the title compound.
ESI-MS: 666.6 [M+Na]⁺ and 644.5 [M+H]⁺ and ¹H and ¹³C NMR data in good agreement with the proposed structure.

### Example 4.

### 6-Morphinyl n-octyl-(β-D-galactopyranosyl)-uronamide acetate.

This analogue is synthesized from the corresponding galacturonic acid intermediate by similar procedures as described in example 3.

### Example 5.

### 3-Morphinyl (β-D-glucopyranosyl) uronamide acetate.

To a 1.50 g of morphine hydrochloride and 0.32 g of lithium hydroxide monohydrate are added in 10 ml of methanol, 2.10 g of tri-O-acetyl-&-D-glucopyranosylbromide uronamide are added. The mixture is stirred at room temperature for 30 min followed by addition of a solution of 0.27 g of lithium hydroxide monohydrate in 10 ml of water. After further 30 min, the pH of the reaction is taken to 8.0 with acetic acid and the unreacted morphine is filtered off. The filtrate is evaporated and the crude residue is purified on a silica gel column using a solvent mixture of ethyl acetate/water/methanol (2:3:1). After lyophylization of the pure fractions from an aqueous acetic acid solution, the compound is isolated as a white solid powder.
ESI-MS: 483.4 [M+Na]⁺ and 461.4 [M+H]⁺ and ¹H and ¹³C NMR data in good agreement with the compound structure.

The starting products may be produced as follows:

### a) Tri-O-acetyl-&-D-glucopyranosyl bromide uronamide.

A solution of 3.00 g of tetra-O-acetyl-β-D-glucopyranosyl uronamide in 15 ml of 30% hydrobromic acid in acetic acid is prepared and kept for 2 days at room temperature until a clear solution is observed. The solvent is evaporated and the residue dissolved in HCCl₃. The solution is extracted with 5% cold aqueous bicarbonate. After drying, the solvent is evaporated and the residue crystallyzed from ethyl acetate/methanol yielding the title compound as a white solid.
M.P. 165-168ºC (desc.), [&]²²_{D} = 219.4 (c=1, HCCl₃). ¹H NMR in accordance with the structural formula.

### b) Tetra-O-acetyl-β-D-glucopyranosyl uronamide.

A mixture of 20 ml of acetic anydride and 15 ml of pyridine is prepared. After cooling to 0ºC, 5.00 g of glucuronamide are added under stirring. The suspension is kept at room temperature under stirring for 48 hr until a clear solution is reached. The solvent is evaporated and the residue crystallized from ethyl acetate/methanol yielding a white solid.
M.P. 153-155ºC, [&]²²_{D} = 112.5 (c=1, HCCl₃).

### Example 6.

### 3-Morphinyl (β-D-galactopyranosyl) uronamide acetate.

The corresponding galactopyranosyl analogue is produced in a similar manner as described in example 5 using the galacturonic acid amide intermediate.

### Example 7.

### 3-Morphinyl (β-D-galactopyranosyl) uronic acid acetate.

The galacturonic acid analogue of the naturally ocurring M3G, is prepared in a similar reaction pathway as described for compound of example 5 starting from a suitable galacturonic acid intermediate.

### Example. 8

### 3-Morphinyl β-D-glucopyranoside acetate.

A suspension of 2.00 g of morphine hydrochloride in 10 ml of methanol is prepared and 0.50 g of lithium hydroxide monohydrate added. After clear solution a new white precicipate appears and 2.55 g of 2,3,4,6 tetra-O-acetyl-&-D-glucopyranosyl bromide are added. The suspension is kept at room temperature under stirring. After 30 min, a solution of 0.60 g of lithium hydroxide monohydrate in 10 ml of water is added. The reaction is allowed to proceed for further 30 min and then is taken to pH:8 with acetic acid. The precipitate of unreacted morphine is filtered off and the filtrate evaporated. The crude product is purified on silica gel column chromatography using a solvent mixture of ethyl acetate/water/methanol (2:3:1). Pooled pure fraction are evaporated, redissolved in water, filtered and lyophilized from an aqueous acetic acid solution yielding the title compound as a white powder.
ESI-MS: 448.4 [M+H]⁺ and 470.5 [M+Na]⁺ and ¹H and ¹³C NMR data in accordance to the proposed structure.

### Example. 9

### 3-Morphinyl β-D-galactopyranoside acetate.

The corresponding galactose derivative is produced in a similar manner following the procedure described in example 8 and using commercial 2,3,4,6 tetra-O-acetyl-&-D-galactopyranosyl bromide.

### Example 10.

### 6-Morphinyl-6'-O-D-galactopyranosyl ether acetate.

A solution of 0.25 g of 6-(3-acetyl)-morphinyl-6'-O-(1:2,3:4 di-O-isopropylidene)-D-galactoplyranosyl ether in 10 ml of methanol is treated with sodium methoxide (0.2 M) for 20 h at room temperature. A neutralization with acetic acid follows. Solvent evaporation yields a residue which is redissolved in 15 ml of a methanol/water mixture. Amberlite IR-120 (H⁺) is added and the reaction is left to proceed for 90 min at room temperature. After filtration, the solvents are evaporated off and the residue redissolved in aqueous acetic acid and lyophilized. The white solid is characterized as corresponding to the title compound.
ESI-MS: 448.3 [M+H]⁺ and 470.4 [M+Na]⁺ and ¹H and ¹³C NMR data in accordance to the structural features of the expected compound.

The intermediate products can be produced as follows:

### a) 6-(3-Acetyl)-morphinyl-6'-O-(1:2,3:4 di-O-isopropylidene)-D-galactoplyranosyl ether.

To a stirred solution of 103 µl of triflic anhydride in 10 ml of dry methylene chloride, a second solution of 0.20 g of 3-acetyl morphine and 50 µl of pyridine in 2 ml of methlene chloride is added dropwise. During the addition, the reaction temperature is kept at 0ºC in an ice bath and later left to reach room temperature. After 30 min 0.50 g of calcium carbonate and a third solution of 0.16 g of 1:2,3:4 di-O-isopropylidene-D-galactopyranose in 3 ml of methylene chloride are added in turn. The reaction mixture is kept overnight under stirring. After filtration and solvent evaporation the residue is suspended in ethyl acetate and washed with water. The organic layer is dryed over anhydrous sodium sulfate, filtered and evaporated. The crude oily residue is purified by column chromatography on silica gel using a chloroform/methanol solvent system (9:1). A pure white amorphous solid is isolated.
ESI-MS: 570.6 [M+H]⁺ and 592.3 [M+Na]⁺ and ¹H and ¹³C NMR data according to the proposed structure.

### Example. 11

### 6-Morphinyl-3'-O-glucofuranosyl ether acetate.

The corresponding glucose derivative is made in a similar way as described in example 10 by using the commercially available 1:2,5:6-di-O-isopropilydene &-D-glucofuranose intermediate.

### Example. 12

### 6-Morphinyl-3'-O-allofuranosyl ether acetate.

This analogue is prepared in a similar manner as described in example 10 from the commercial acetone derivative of allose: 1:2,5:6-di-O-isopropilydene &-D-allofuranose.

### Example. 13

### 6-Morphinyl-6'-O-glucopyranosyl ether acetate.

This derivative is prepared by an analogous procedure as described in example 10 using the commercially available 1:2,3:4 tetra-O-acetyl-β-D-glucopyranose by avoiding the Amberlite IR-120 treatment.

### Example. 14

### 6-Morphinyl-2'-O-mannopyranosyl ether acetate.

The mannosyl analogue is also prepared following the synthetic route described above (Example 10) from 1:3,4:6 tetra-O-acetyl-β-D-mannopyranose avoiding the step of acetonide hydrolysis by Amberlite IR-120.

### Example 15.

### 6-Morphinyl-β-D-glucopyranoside acetate.

To a solution of 0.40 g of 6-(3-acetyl)-morphinyl-β-D-glucopyranoside tetraacetate in 12 ml of dry methanol, 0.25 ml of 30% sodium methoxide in methanol are added. After 30 minutes at room temperature under stirring, the solution is neutralized with acetic acid. The solvent is removed off and the crude residue is purified by column chromatography on silica gel using as a solvent ethyl acetate/water/methanol (2:3:1). After pooling the pure fractions and solvent evaporation followed by lyophylization from an aqueous acetic acid solution, the title compound is isolated as a white pure solid.
ESI-MS: 448.3 [M+H]⁺ and 470.2 [M+Na]⁺ and ¹H and ¹³C NMR spectral data in good agreement to the proposed structure.

The intermediate products can be prepared as follows:

### a) 6-(3-O-Acetyl)-morphinyl-β-D-glucopyranoside tetraacetate.

Under argon atmosphere a suspension of 0.32 g of 3-O-acetyl morphine and 0.25 g of silver triflate in 10 ml of dry dichloromethane in the presence of 3Å molecular sieves is prepared and kept under stirring at -20ºC. The reaction is effected by dropwise addition of 0.40 g of tetraacetyl-&-D-glucopyranosyl bromide in 5 ml of dry dichloromethane. The reaction is left to proceed for 3 h and 0.15 ml of triethylamine are added and the mixture is allowed to warm up to room temperature. The silver salts are filtered off and the filtrate is evaporated under reduced pressure. The residue is finally purified on a silica gel bed eluted with a chloroform/methanol mixture (9:1) yielding the title compound.
ESI-MS: 658.5 [M+H]⁺ and 680.6 [M+Na]⁺ and ¹H and ¹³C NMR spectral data in accordance to the proposed structure.

### Example. 16

### 6-Morphinyl-β-D-galactopyranoside acetate.

The corresponding galactose derivative is achieved in a similar way as described in example 15 by using the acetobromo galactose intermediate.

### Example 17.

### 6-Morphinyl (β-D-glucopyranosyl) uronamide acetate.

The glucuronic acid amide derivative is prepared following a similar procedure as described for example 15 using the corresponding acetobromo compounds.

### Example 18.

### 6-Morphinyl (β-D-galactopyranosyl) uronamide acetate.

Similar procedure as described in example 15 leds to this analogue provided the corresponding galacturonic acid amide is used.

### Example 19.

### 6-Morphinyl (β-D-galactopyranosyl) uronic acid acetate.

The corresponding galacturonic acid analogue of the naturally occurring M6G is prepared in a similar manner as in the reaction course described for example 15 starting from a suitable galacturonic acid intermediate.

### Example 20.

### 3-Naloxonyl-β-D-gluconyranoside acetate.

To a solution of 0.50 g of naloxone hydrochloride and 0.10 g of lithium hydroxide in 5 ml of methanol are added 0.56 g of &-D-glucopyranosyl bromide tetraacetate. After stirring for 30 min at room temperature, a solution of 0.20 g of lithium hydroxide in 5 ml of water is added. The reaction is allowed to proceed for a further 30 min period and then is taken to pH:8 with acetic acid. The unreacted naloxone is filtered off and the filtrate further acidified and evaporated. The residue is finally crystallized from 95% ethanol affording the title compound.
ESI-MS: 490.4 [M+H]⁺ and 512.3 [M+Na]⁺ and ¹H and ¹³C NMR spectral data in accordance to the proposed structure.

### Example. 21

### 3-Naloxonyl-β-D-galactopyranoside acetate.

This analogue is prepared by an analogous procedure using &-D-galactopyranosyl bromide tetraacetate as starting material.

### Example. 22

### 3-Naloxonyl-β-D-glucoppyranosyl uronamide acetate.

The corresponding glucuronic acid amide analogue is synthesized by a similar procedure as described in example 20.

### Example. 23

### 3-Naloxonyl-β-D-galactopyranosyl uronamide acetate.

The galacturonic acid amide derivative is produced in a similar manner as described in example 20.

### Example. 24

### 3-Naloxonyl-β-D-galactopyranosyl uronic acid acetate.

This analogue is prepared by a similar procedure than the one provided in example 20 using a suitable galacturonic acid intermediate.

### Example 25.

### 6-Morphinyl-β-D-glucopyranosyl-thiouretane acetate.

A solution of 0.70 g of 6-(3-O-acetyl)-morphinyl 2,3,4,7 tetra-O-acetyl-β-D-glucopyranosyl thiouretane in 10 ml of absolute methanol is treated with a catalytic quantity of 1N sodium methoxide in methanol. After aproximately 30 min the reaction is completed and the mixture is neutralized with acetic acid. The solvent is evaporated and the crude product purified by silica gel column chromatography using a ethyl acetate/water/methanol (2:3:1) solvent mixture. Pure fractions are pooled evaporated and the residue lyophilized from aqueous acetic acid to yield the title compound as a white lyophilizate powder.
ESI-MS: 507.6 [M+H]⁺ and 529.4 [M+Na]⁺ and ¹H and ¹³C NMR spectral data in good agreement to the structure of title compound.

The intermediate product can be prepared as follows:

### a) 6-(3-O-Acetyl)-morphinyl 2,3,4,6 tetra-O-acetyl-β-D-glucopyranosyl thiouretane.

To a solution prepared by dissolving 0.42 g of 3-O-acetyl morphine in 30 ml of acetonitrile/water (3:1), 0.5 g of 2,3,4,6 tetra-O-acetyl-β-D-glucopyranosyl isothiocianate are added. The reaction is stirred at room temperature for about 1 h. The solvent is then evaporated in vacuo and the residue purified on silica gel eluted with a chloroform/methanol (8:2) mixture yielding the corresponding title compound.
ESI-MS: 717.5 [M+H]⁺ and 739.4 [M+Na]⁺ and ¹H and ¹³C NMR spectral data in good agreement to the proposed structure.

### Example. 26

### 6-Codeinyl-β-D-glucopyranoside acetate.

To a stirred solution kept at room temperature and under argon atmosphere, of 1.15 g of 6-codeinyl-2,3,4,6-O-tetraacetate-β-D-glucopyranoside, in 40 mL of dry MeOH, 0.27 g of sodium methoxide are added in portions keeping the pH between 7 and 8. After 1 hour the reaction is completed and the solvent removed under vacuum. The crude residue is purified on a silica gel column eluted with a solvent mixture of ethyl acetate/water/methanol (3:2:1). After this procedure, lyophilization of the pure fraction from an aqueous acetic acid solution affords the title compound as a white solid.
ESI-MS: 462.4 [M+H]⁺ and 484.3 [M+Na]⁺ and ¹H and ¹³C NMR spectral data in accordance to the structure of title compound.

The starting product can be produced as follows.

### a) 6-Codeinyl-2,3,4,6-O-tetraacetate-β-D-glucopyranoside.

Under Argon atmosphere a solution of 2.99 g of codeine and 3.12 g of β-D-glucose pentaacetate in 60 ml of dry acetonitrile, 6 ml of borontrifluoride ethyl etherate complex are added dropwise. The mixture is stirred at 0°C for 30 min and then is kept overnight at room temperature. After adding 100 ml of chloroform the solvent is evaporated. The crude residue is dissolved with 100 ml of water and extracted with ethyl ether and chloroform. The chloroform layer is washed with saturated solution of NaCl and the solvent evaporated to dryness. After redissolving the crude residue the aqueous solution is adjusted to pH=7 and extracted again with chloroform. The organic layer is evaporated to dryness yielding the title compound.
ESI-MS: 630.6 [M+H]⁺ and 652.4 [M+Na]⁺ and ¹H and ¹³C NMR spectral data in good agreement to the structural features of the title compound.

### Example. 27

### 6-Codeinyl β-D-galactopyranoside acetate.

Following an analogous procedure as described for example 26 the galactoside derivative of codeine is prepared from β-D galactose pentaacetate.

### Example. 28

### 6-Codeinyl-(β-D-gluconyranosyl-uronic acid acetate.

To a stirred solution prepared by dissolving 1g of 6-codeinyl-β-D-glucopyranoside described in example 26, 87 mg of sodium bromide and 2,2 mg of TEMPO in 55 ml of water, 3.7 mL of 10% sodium hypochlorite are added. The reaction temperature is kept at 0ºC and the pH is adjusted at 10.8 by addition of diluted sodium hydroxide solution. After 1 hour, the reaction is quenched by addition of ethanol and then neutralized with diluted HCl. After concentration of the reaction mixture to dryness, the residue is purified on a silica gel column eluted with a solvent mixture of ethyl acetate/water/methanol (2:3:1). In this way, lyophilization from an aqueous acetic acid solution yields the title compound as a white lyophilizate powder.
ESI-MS: 476.3 [M+H]⁺ and 498.5 [M+Na]⁺ and ¹H and ¹³C NMR spectral data in good agreement to the structure of title compound.

### Example. 29

### 6-Codeinyl (β-D-galactopyranosyl) uronic acid acetate.

A similar reaction described for example 28 allows to synthesize the galacturonic acid analogue of codeine from the compound described in example 27.

### Example. 30

### 6-Codeinyl n-octyl (β-D-glucopyranosyl) uronamide acetate.

To a solution of 0.30 g of 6-codeinyl (β-D-glucopyranosyl) uronic acid acetate prepared as described in example 28, 80 mg of n-octylamine and 0.10 g of HOBt in 15 ml of DMF, a second solution of 0.24 g of HBTU in 10 ml of DMF are added. The reaction is kept overnight under stirring at room temperature. After solvent evaporation, the crude product is purified by column chromatography on silica gel using as solvent a mixture of ethyl acetate/water/methanol (9/4/5). The title compound is isolated as a white lyophilizate from an aqueous acetic acid solution.
ESI-MS: 609.7 [M+Na]⁺ and 587.5 [M+H]⁺, ¹H and ¹³C NMR data in accordance with the expected structure.

### Example. 31

### 6-Codeinyl n-octyl (β-D-galactopyranosyl) uronamide acetate.

Starting from the compound described in example 29 and following an analogous description as for example 30, the galacturonic acid derivative is prepared.

### Example. 32

### 4-Loperamidyl-β-D-glucopyranoside acetate.

To a solution of 0.45 g of 4-loperamidyl 2,3,4,6 tetra O-acetyl-β-D glucopyranoside in 12 ml of dry methanol, 0.25 ml of 30% sodium methoxide in methanol are added. After 30 minutes at room temperature under stirring, the solution is neutralized with acetic acid. The solvent is removed under reduced pressure and the crude residue is purified by column chromatography on silica gel using as a solvent ethyl acetate/water/methanol (2:3:1). After collection of pure samples and lyophilization from an aqueous acetic acid solution, the title compound is isolated.
ESI-MS: 640.0 [M+H]⁺ and 662.4 [M+Na]⁺ and ¹H and ¹³C NMR spectral data in good agreement to the proposed structure.

The intermediate products can be prepared as follows:

### a) 4-Loperamidyl 2,3,4,6 tetra O-acetyl-β-D-gluconyranoside.

Under Argon atmosphere a solution of 0.50 g of loperamide and 0.27 g of silver triflate in 10 ml of dry dichloromethane in the presence of 3Å molecular sieves is prepared and kept under stirring at -20ºC. The reaction is started by dropwise addition of 0.43 g of tetraacetyl-&-D-glucopyranosyl bromide in 5 ml of dry dichloromethane. The reaction is left to proceed for 3 h and 0.10 ml of triethylamine are added and the mixture is allowed to warm up to room temperature. The silver salts are filtered off and the filtrate is evaporated under reduced pressure. The residue is finally purified on a silica gel column eluted with a chloroform/methanol mixture (9:1) yielding the title compound.
ESI-MS: 808.3 [M+H]⁺ and 830.1 [M+Na]⁺ and ¹H and ¹³C NMR spectral data in accordance to the structural features of this compound.

### Example. 33

### 4-Loperamidyl-β-D-galactopyranoside acetate.

The title compound is prepared in analogous fashion from the galactobromo sugar as starting material.

### Example. 34

### 4-Loperamidyl-β-D-galactopyranosyl uronic acid acetate.

The galacturonic acid analogue is synthesized according to a similar process described in example 32.

### Example. 35

### 4-Loperamidyl-β-D-glucopyranosyl uronamide acetate.

The corresponding glucuronamide derivative is also produced by following a reaction pathway as described above.

### Example. 36

### 4-Loperamidyl-β-D-galactopyranosyl uronamide acetate.

Taking as a reference the synthetic route of example 32, this derivative is also prepared using the appropriate bromosugar intermediate.

### Example. 37

### Methadyl-β-D-glucopyranoside acetate.

To a solution of 1.00 g of methadyl 2,3,4,6 tetra O-acetyl-β-D glucopyranoside in 20 ml of dry methanol, 0.5 ml of 30% sodium methoxide in methanol are added. After 30 minutes at room temperature under stirring, the solution is neutralized with acetic acid. The solvent is removed under reduced pressure and the crude residue is purified by column chromatography on silica gel using as a solvent ethyl acetate/water/methanol (2:3:1). Pure fractions are collected which render the title compound after lyophilization from an aqueous acetic acid solution.
ESI-MS: 473.6 [M+H]⁺ and 495.8 [M+Na]⁺ and ¹H and ¹³C NMR spectral data in good agreement to the proposed structure.

The intermediate products can be prepared as follows:

### a) Methadyl 2,3,4,6 tetra O-acetyl-β-D-glucopyranoside.

Under Argon atmosphere a solution of 0.80 g of methadol and 0.66 g of silver triflate are dissolved in 15 ml of dry dichloromethane in the presence of 3Å molecular sieves The mixture is prepared and kept under stirring at -20ºC. The reaction is started by dropwise addition of a solution of 1.00 g of tetraacetyl-&-D-glucopyranosyl bromide in 10 ml of dry dichloromethane. The reaction is left to proceed for 3 h and 0.26 ml of triethylamine are added and the mixture is allowed to warm up to room temperature. The silver salts are filtered off and the filtrate is evaporated under reduced pressure. The residue is finally purified on a silica gel column eluted with a chloroform/methanol mixture (8:2) yielding the title compound.
ESI-MS: 641.7 [M+H]⁺ and 663.8 [M+Na]⁺ and ¹H and ¹³C NMR spectral data in accordance to the structural characteristics of this compound.

### b) Methadol.

To a 20 ml ethanol solution of 1 g of methadone hydrochloride, 0.40 g of sodium borohydride are added. The reaction is kept at room temperature for 2 h. The solvent is evaporated and the residue suspended in ethyl acetate. After washing the organic layer with agueous 10% bicarbonate solution and drying with anhydrous sodium sulfate, the title compound is isolated. ESI-MS 333.4 [M+Na]⁺

### Example. 38

### Methadyl-β-D-galactopyranoside acetate.

This compound is produced by an analogous manner from the bromosugar as starting material.

### Example. 39

### Methadyl-β-D-galactopyranosyl uronic acid acetate.

The analogue of galacturonic acid is prepared according to a similar procedure as described in example 37.

### Example. 40

### Methadyl-β-D-glucopyranosyl uronamide acetate.

The corresponding glucuronamide derivative is also synthesized by following a reaction sequence as described above.

### Example. 41

### Methadyl-β-D-galactopyranosyl uronamide acetate.

By a similar synthetic route of example 37, this derivative is also prepared using the appropriate bromosugar intermediate.

### Example. 42

### 6-Pentazocyl-β-D-glucopyranoside acetate.

To a solution of 1.00 g of pentazocine and 0.12 g of lithium hydroxide in 10 ml of methanol are added 1.45 g of &-D-glucopyranosyl bromide tetraacetate. After stirring for 30 min at room temperature, a solution of 0.40 g of lithium hydroxide in 10 ml of water is added. The reaction is allowed to proceed for a further 30 min period and then is taken to pH:8 with acetic acid. The unreacted pentazocine is filtered off and the filtrate further purified by silica gel column chromatrography using as a solvent system ethyl acetate/water/methanol (1:1:1) affording the title compound which is then lyophilized from an aqueous acetic acid solution.
ESI-MS: 448.4 [M+H]⁺ and 470.3 [M+Na]⁺ and ¹H and ¹³C NMR spectral data in accordance to the proposed structure.

### Example. 43

### 6-Pentazocyl-β-D-galactopyranoside acetate.

By an analogous procedure as described for example 42 the title compound is prepared using the corresponding &-D-galactopyranosyl bromide tetraacetate.

### Example. 44

### 6-Pentazocyl-β-D-galactopyranosyl uronic acid acetate.

This analogue is prepared by a similar route as described in example 42 starting from the methyl (tri-O-acetyl-&-D-galactopyranosyl bromide)-uronate intermediate.

### Example. 45

### 6-Pentazocyl-β-D-glucopyranosyl uronamide acetate.

The corresponding glucuronamide analogue is prepared by a similar way from the acetobromo sugar intermediate.

### Example. 46

6-Pentazocyl-β-D-galactopyranosyl uronamide acetate. The galacturonamide derivative is synthesized following an analogous route as described in example 42.

### Example. 47

### 6-Cyclazocyl-β-D-glucopyranoside acetate.

To a solution of 0.50 g of cyclazocine and 0.06 g of lithium hydroxide in 5 ml of methanol are added 0.75 g of &-D-glucopyranosyl bromide tetraacetate. After stirring for 30 min at room temperature, a solution of 0.20 g of lithium hydroxide in 5 ml of water is added. The reaction is allowed to proceed for a further 30 min period and then is taken to pH:8 with acetic acid. The unreacted cyclazocine is filtered off and the filtrate further purified by silica gel column chromatrography using as a solvent system ethyl acetate/water/methanol (1:1:1) affording the title compound which is finally lyophilized from an agueous acetic acid solution.
ESI-MS: 434.3 [M+H]⁺ and 456.6 [M+Na]⁺ and ⁺H and ¹³C NMR spectral data in accordance to the structure of title compound.

### Example. 48

### 6-Cyclazocyl-β-D-galactopyranoside acetate.

The galactoside is similarly prepared as described in example 37 using the corresponding bromoacetate derivative.

### Example. 49

### 6-Cyclazocyl-β-D-galactopyranosyl uronic acid acetate.

This derivative is prepared using the corresponding uronate following similar procedures already described.

### Example. 50

### 6-Cyclazocyl-β-D-glucopyranosyl uronamide acetate.

Following an analogous recipy as described in example 47, the glucuronamide analogue is prepared.

### Example. 51

### 6-Cyclazocyl-β-D-galactopyranosyl uronamide acetate.

A similar procedure as describe previously leads to this derivative.

### Biological activity.

The compounds of the invention show pharmacological activity and therefore are indicated for use as pharmaceuticals for therapeutic purposes.

The activity of the compounds of the invention may be observed in either "in vivo" or "in vitro" standard biological tests. In general terms, the compounds of the invention show equal or superior potency than their unmodified opiate counterparts (i.e., the corresponding sugar-free opiates) when administered intraperitoneally in laboratory animals. Some of them also exhibit longer duration of action. In accordance, the compounds of the invention are therefore indicated for the treatment of the same disorders as those of the unmodified opiates.

The compounds of the invention may be compared with the unmodified parent opiates in standard antinociceptive tests, i.e. in terms of potency and duration of action. For example, phamacological tests may be effected to examine the effects of the compounds of the invention on suppressing pain associated to nocius stimuli induced in laboratory animals. Thus, these compounds can be tested by measuring the time treated animals are insensitive to a given painful stimuli.

In spite of the different classes among the compounds of the invention which can be appreciated in accordance to their chemical structure, all are pain-related substances and therefore can be examined on the tail immersion and paw pressure tests as follows.

Male Sprague-Dawley rats weighing 200 g are used. They are housed singly in a room with controlled temperature 21ºC on a 12h light-dark cycle and given free access to food and water.

The tail flick test, is conducted as described by D'Amour F.E. and Smith D.L. in *A method for determining loss of pain sensations*, J.Pharmacol. Exp. Ther. 72,74, 1941.

Mechanical nociceptive thresholds are determined by the paw pressure test on the left hind paw as described in the literature (Randall L.O. and Selito J.J. *A method for measurement of analgesic activity on inflamed tissue*, Arch. Int. Pharmacodyn. Ther. 1957, 11, 409-419.). Pain thresholds are measured with an analgesymeter. The apparatus is set up to apply a force of 0-1000 g increasing from zero at a rate of 64 g/s. The nociceptive threshold is taken as the point at which the rat vocalize or struggle vigorously.

In the above described tests, the following results obtained with example 1 compound are given as a representative example. Morphine and M3G have been used as reference compounds. After intraperitoneal administration of a dosis of 0.1 mg/Kg of example 1 compound, a maximal antinociceptive response was observed, this is a 100% of analgesia was measured. On the same set of experiments and by the same administration route, a morphine dosis of 5 mg/Kg induced a 65-75% of analgesia while a dosis of 1 mg/Kg of M3G showed the same effect.

Results provided by compound of example 26 illustrate a different case where the incorporation of the sugar moiety into the opiate molecule does not affect the potency of the parent compound. Thus, in the above described tests, an intraperitoneally administered dosis of 5 mg/Kg of codeine induces the same degree of analgesia as compound of example 26.

## Claims

1. A sugar derivative of a biologically active opiate which has an enhaced analgesic potency if compared to the non-sugar modified opiate and carries at least one sugar residue which is attached by direct O-glycosidic bond or ether coupling to one hydroxyl group of the opiate or by means of a C-glycosidic bond between two other suitable functions on the two constituting moieties.

2. A derivative according to claim 1 wherein the derivative contains more than one sugar residue.

3. A derivative according to claim 1 and 2 wherein the derivative contains more than one opiate molecule per sugar residue.

4. A sugar derivative of biologically active opiates of the following formulae:
a) 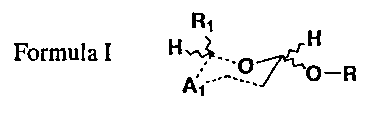 wherein 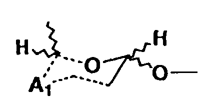 is a glycosyl residue being linked via glycosidic bond to an hydroxyl group of a biologically active opiate, R₁ is hydrogen, hydroxyl group, a saturated or insaturated or aromatic linear or branched or cyclic alcohol and/or acid residue with 1 to 18 C-atoms, a sulfate(s) or phosphate(s) residue(s) and R is the residue of a biologically active opiate of formula R-OH, wherein the OH group is either an alcohol or a phenol function.
b) 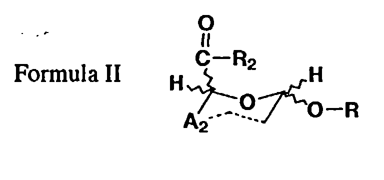 wherein 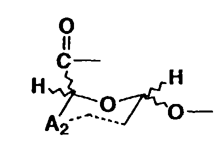 is a residue of an uronic acid, R₂ is hydroxyl, amino, a saturated or insaturated or aromatic linear or branched or cyclic amine or alcohol residue with 1 to 18 C-atoms and R is the residue of a biologically active opiate of formula R-OH, wherein the OH group is either an alcohol or a phenol function.
c)
A₃-O-R Formula III
wherein A₃-O- is a glycosyl residue being linked via ether bond to an hydroxyl group of a biologically active opiate and R is the residue of a biologically active opiate of formula R-OH, wherein the OH group is either an alcohol or a phenol function.
d) 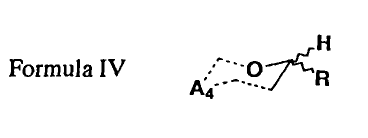 wherein 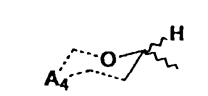 is a deoxy glycosyl residue being coupled via C-C single bond to an atom carbon of a biologically active opiate and R is the residue of a biologically active opiate.
e) 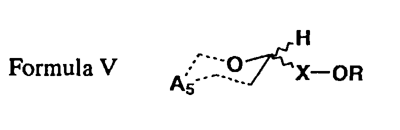 wherein 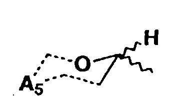 is a sugar residue, R denotes the residue of a biologically active opiate of formula R-OH and X is a linker group, i.e., NHCO or NHCS coupling the opiate to the sugar residue.
Acid salts and complexes of these opiate glycoconjugates of formula I to V are also among this series of compounds.

5. A compound of claim 4 containing one sugar moiety.

6. A compound of claim 4 containing two sugar moieties.

7. A compound of claim 4 containing three sugar moieties.

8. A compound according to claim 4 to 7 containing more than one opiate molecule per sugar residue.

9. A compound of any of claims 4 to 8 which is a compound of Formula I.

10. A compound of anyone of claims 1 to 8 presenting in its molecule a sugar residue according to the formula below. 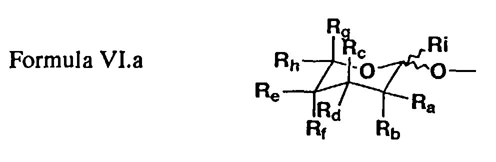 wherein one of the radicals Rₐ and R_{b} is hydrogen and the other is R₁ or NH₂ or NHOCOCH₃, one of the radicals R_{c} and R_{d} is hydrogen and the other is R₁ or O-glycosyl, being the glycosyl radical derived from a reducing mono-, di- or oligossacharide, one of the radicals, Rₑ and R_{f} is hydrogen and the other is R₁ or O-glycosyl, being the glycosyl radical derived from a reducing mono-, di- or oligossacharide, one of the radicals R_{g} and Rₕ is hydrogen and the other is CH₂-R₁ or O-glycosyl, being the glycosyl radical derived from a reducing mono-, di- or oligossacharide, the radical Rᵢ is hydrogen or CH₂OH, and the radical R₁ as described above.

11. A compound according to claim 10 where a sugar residue is chosen from the following radicals: glucosyl, galactosyl, mannosyl, fucosyl, xylosyl, rhamnosyl, glucosaminyl, N-acetylglucosaminyl, octylglucopyranosyl, ciclohexylglucopyranosyl, benzylglucopyranosyl, glucosyl sulfate, glucosyl phosphate, glucosaminyl sulfate, N-acetyl-glucosaminyl sulfate, lactosyl, gentiobiosyl, chitobiosyl, N-acetyl-lactosaminyl, cellobiosyl, maltosyl, melibiosyl and L-sorbosyl.

12. A compound according to claims 1 to 8 presenting in its molecule a sugar residue according to the formula below. 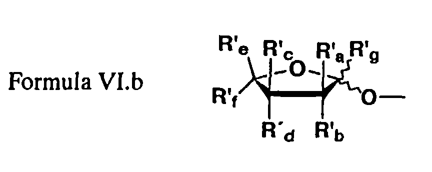 weherein one of the radicals R'ₐ and R'_{b} is hydrogen and the other is R₁ or O-glycosyl, being the glycosyl radical derived from a reducing mono-, di- or oligosaccharide, one of the radicals R'_{c} aand R'_{d} is hydrogen and the other is R₁ or O-glycosyl, being the glycosyl radical derived from a reducing mono-, di- or oligosaccharide, one of the radicals R'ₑ and R'_{f} is hydrogen and the other is CH₂ or CHOH-CH₂OR₁ or O-glycosyl, being the glycosyl radical derived from a reducing mono-, di- or oligosaccharide, the radical R'_{g} is hydrogen or CH₂OH and the radical R₁ as described above.

13. A compound according to claim 12 where a sugar residue is selected from the following radicals: D-ribosyl, D-arabinosyl, D- xylosyl, D-lyxosyl, 2-deoxy-D-ribosyl, altosyl, idosyl, allosyl, D-fructosyl, D-sicosyl, D-sorbosyl, D-tagatosyl, D-xylulosyl, D-ribulosyl, D-glucofuranosyl, D-galactofuranosyl, octylribofuranosyl, cyclohexylxylofuranosyl, benzylarabinofuranosyl and D-fructosyl fosfate.

14. A compound according to anyone of claims 4 to 8 which is of Formula II.

15. A compound of anyone of claims 1 to 8 presenting in its molecule a sugar residue according to the formula below. 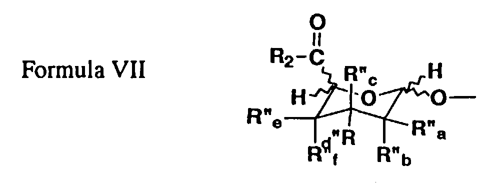 wherein one of the radicals R''ₐ and R''_{b} is hydrogen and the other is hydrogen, hydroxyl, amino or acetylamino group, one of the radicals R''_{c} and R''_{d} is hydrogen and the other is hydrogen or O-glycosyl, being the glycosyl radical derived from a reducing mono-, di- or oligossaccharide, one of the radicals R''ₑ and R''_{f} is hydrogen and the other is hydrogen or O-glycosyl, being the glycosyl radical derived from a reducing mono-, di- or oligossaccharide and the radical R₂ as defined above.

16. A compound according to claim 15 where a sugar moiety is selected from the following sugar derivatives of the uronic acid series: glucuronic acid, galacturonic acid, glucuronamide, glucosaminuronic acid, octylgalacturonamide, cyclohexylgalacturonate, decylglucoronate and benzylglucoronamide or other complex sugar acids such as muramic acid, neuraminic acid, acetyl muramic, acetyl neuraminic and sialic acid.

17. A compound of Formula III as defined in anyone of claims 4 to 8, 10 to 13, 15 and 16.

18. A compound of Formula IV as defined in anyone of claims 4 to 8, 10 to 13, 15 and 16.

19. A compound of Formula V as defined in anyone of claims 4 to 8, 10 to 13, 15 and 16.

20. A compound of claim 19 wherein X is NHCO or NHCS.

21. A compound of anyone of claims 1 to 20 wherein the opiate is a derivative of morphinan structure.

22. A sugar derivative of compound of formula VIII. 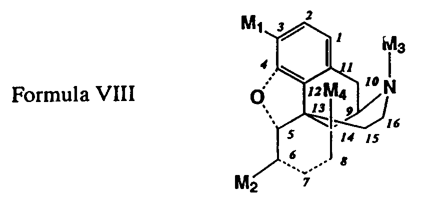 wherein, M₁ is hydroxyl, methoxy, ethoxy, acetoxy or 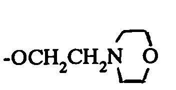 group; M₂ is hydrogen, hydroxyl, oxy, acetoxy, methoxy and methene group; M₃ is methyl, methylen cyclopropyl, methylen cyclobutyl and allyl group; M₄ hydrogen or hydroxyl; the bond between carbon atoms in positions 6-7 and 7-8 can be either single or double; the ether function between position 4 and 5 can be either present or not present on the molecule. Compounds of formula VIII can either exist in the form of levo or dextrorotatory isomers as well as salts and complexes.

23. A sugar derivative of claim 22 of formula

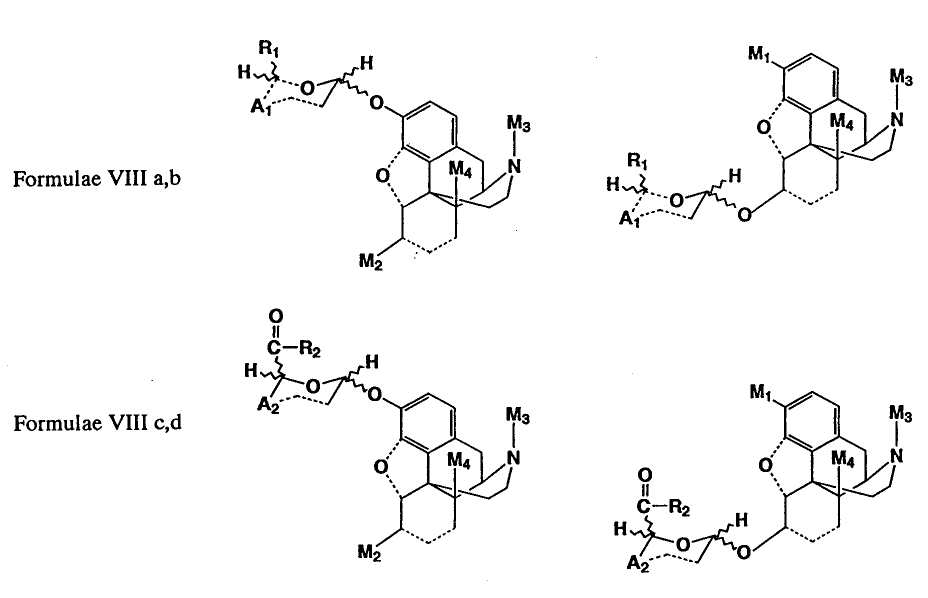

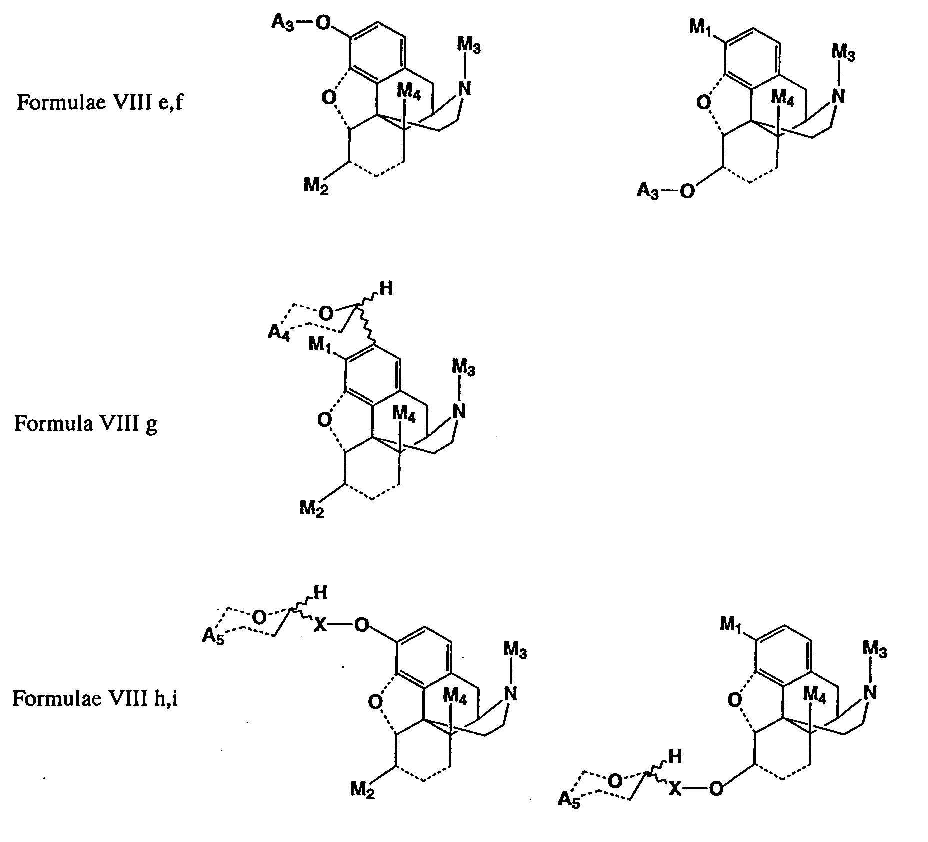

24. A compound of claim 23 characterized by the feature that the radicals: 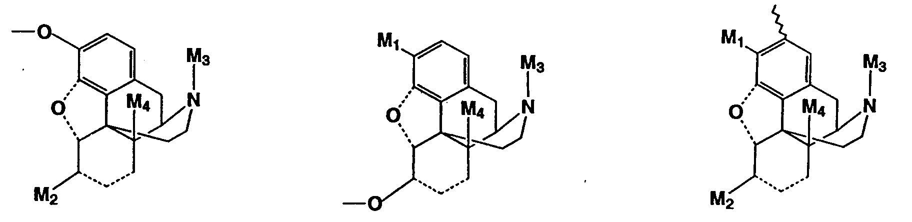 correspond to the morphine structure.

25. A compound of claim 24 which is 3-morphinyl n-octyl-(β-D-glucopyranosyl) uronamide.

26. A compound of claim 24 which is 3-morphinyl n-octyl-(β-D-galactopyranosyl) uronamide.

27. A compound of claim 24 which is 6-morphinyl n-octyl-(β-D-glucopyranosyl) uronamide.

28. A compound of claim 24 which is 6-morphinyl n-octyl-(β-D-galactopyranosyl) uronamide.

29. A compound of claim 24 which is 3-morphinyl (β-D-glucopyranosyl) uronamide.

30. A compound of claim 24 which is 3-morphinyl (β-D-galactopyranosyl) uronamide.

31. A compound of claim 24 which is 3-morphinyl (β-D-galactopyranosyl) uronic acid.

32. A compound of claim 24 which is 3-morphinyl β-D-glucopyranoside.

33. A compound of claim 24 which is 3-morphinyl β-D-galactopyranoside.

34. A compound of claim 24 which is 6-morphinyl-6'-O-D-galactopyranosyl ether.

35. A compound of claim 24 which is 6-morphinyl-3'-O-D-glucofuranosyl ether.

36. A compound of claim 24 which is 6-morphinyl-3'-O-D-alloluranosyl ether.

37. A compound of claim 24 which is 6-morphinyl-6'-O-D-glucopyranosyl ether.

38. A compound of claim 24 which is 6-morphinyl-2'-O-D-mannopyranosyl ether.

39. A compound of claim 24 which is 6-morphinyl β-D-glucopyranoside.

40. A compound of claim 24 which is 6-morphinyl β-D-galactopyranoside.

41. A compound of claim 24 which is 6-morphinyl (β-D-glucopyranosyl) uronamide.

42. A compound of claim 24 which is 6-morphinyl (β-D-galactopyranosyl) uronamide.

43. A compound of claim 24 which is 6-morphinyl (β-D-galactopyranosyl) uronic acid.

44. A compound of claim 24 which is 6-morphinyl β-D-glucopyranosyl thiouretane.

45. A compound of claim 23 which is 3-naloxonyl-β-D-glucopyranoside.

46. A compound of claim 23 which is 3-naloxonyl-β-D-galactopyranoside.

47. A compound of claim 23 which is 3-naloxonyl-β-D-glucopyranosyl uronamide.

48. A compound of claim 23 which is 3-naloxonyl-β-D-galactopyranosyl uronamide.

49. A compound of claim 23 which is 3-naloxonyl-β-D-galactopyranosyl uronic acid.

50. A compound of claim 23 which is 6-codeinyl-β-D-glucopyranoside.

51. A compound of claim 23 which is 6-codeinyl-β-D-galactopyranoside.

52. A compound of claim 23 which is 6-codeinyl (β-D-glucopyranosyl) uronic acid.

53. A compound of claim 23 which is 6-codeinyl (β-D-galactopyranosyl) uronic acid.

54. A compound of claim 23 which is 6-codeinyl n-octyl (β-D-glucopyranosyl) uronamide.

55. A compound of claim 23 which is 6-codeinyl n-octyl (β-D-galactopyranosyl) uronamide.

56. A compound of anyone of claims 1 to 20 wherein the opiate is a derivative of meperidine structure.

57. A sugar derivative of compound of formula IX. 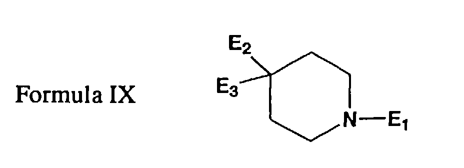 wherein E₁ is methyl, phenylethyl, p-phenylethylamine, -(CH₂)₃-NH-C₆H₅, -(CH₂)₂-C(C₆H₅)₂-CN, -(CH₂)₂-C(C₆H₅)₂-CO-N(CH₃)₂, 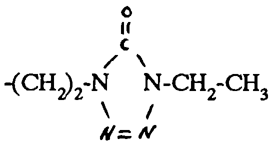 and 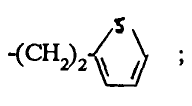 E₂ is hydrogen, phenyl, m-hydroxyphenyl, p-chlorophenyl, methylenmetoxide and piperidinyl; E₃ is hydroxyl, ethylcarbonyl, carboxamide, ethyloxycarbonyl, ethylcarboxyl and -N(C₆H₅)-CO-CH₂-CH₃.

58. A sugar derivative of claim 57 of formula 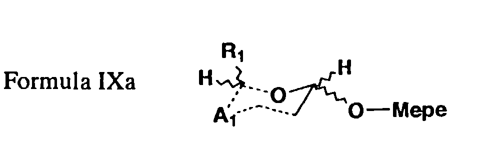 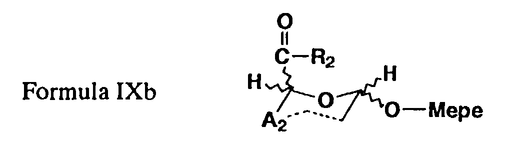
A₃-O-Mepe Formula IXc 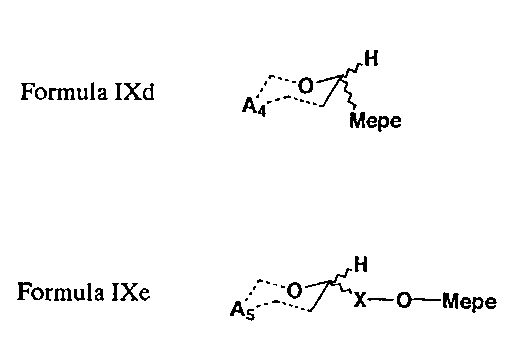
where **Mepe** denotes the residue of meperidine or of a derivative or analogue as defined in claim 57.

59. A compound of claim 58 which is 4-loperamidyl-β-D-glucopyranoside acetate.

60. A compound of claim 58 which is 4-loperamidyl-β-D-galactopyranoside acetate.

61. A compound of claim 58 which is 4-loperamidyl-β-D-galactopyranosyl uronic acid acetate.

62. A compound of claim 58 which is 4-loperamidyl-β-D-glucopyranosyl uronamide acetate.

63. A compound of claim 58 which is 4-loperamidyl-β-D-galactopyranosyl uronamide acetate.

64. A compound of anyone of claims 1 to 20 wherein the opiate is a derivative of methadone structure.

65. A sugar derivative of compound of formula X. 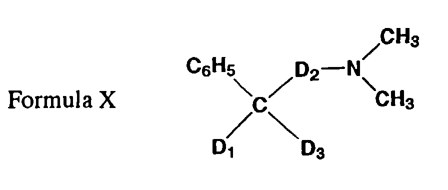 wherein D₁ is phenyl or benzyl; D₂ is -CH₂-CH(CH₃)-, -CH(CH₃)-CH₂- and -(CH₂)₂-; D₃ is ethylcarbonyl, ethylcarboxyl and -CH(OCOCH₃)-CH₂-CH₃.

66. A sugar derivative of claim 65 of formula 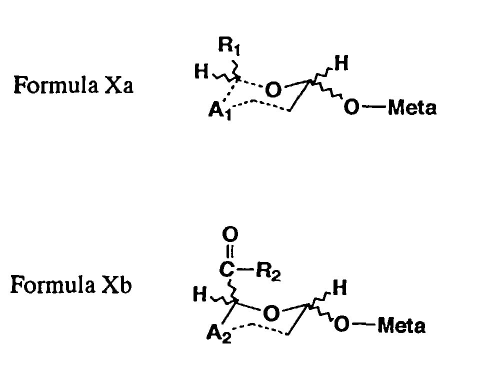
A₃-O-Meta Formula Xc 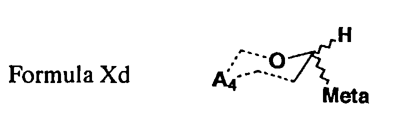 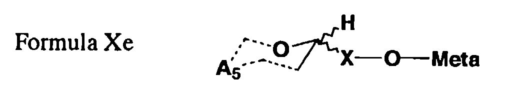
where **Meta** refers to the residue of methadone or of a derivative or analogue as defined in claim 65.

67. A compound of claim 66 which is methadyl-β-D-glucopyranoside acetate.

68. A compound of claim 66 which is methadyl-β-D-galactopyranoside acetate.

69. A compound of claim 66 which is methadyl-β-D-galactopyranosyl uronic acid acetate.

70. A compound of claim 66 which is methadyl-β-D-glucopyranosyl uronamide acetate.

71. A compound of claim 66 which is methadyl-β-D-galactopyranosyl uronamide acetate.

72. A compound of anyone of claims 1 to 20 wherein the opiate is a derivative of benzomorphane structure.

73. A sugar derivative of compound of formula XI. 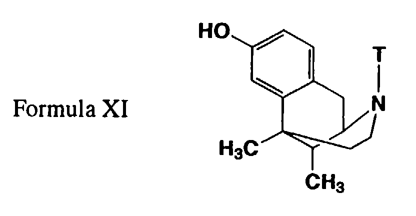 wherein T is methyl, phenylethyl, methylcyclopropyl and -CH₂-CH=C(CH₃)₂

74. A sugar derivative of claim 73 of formula

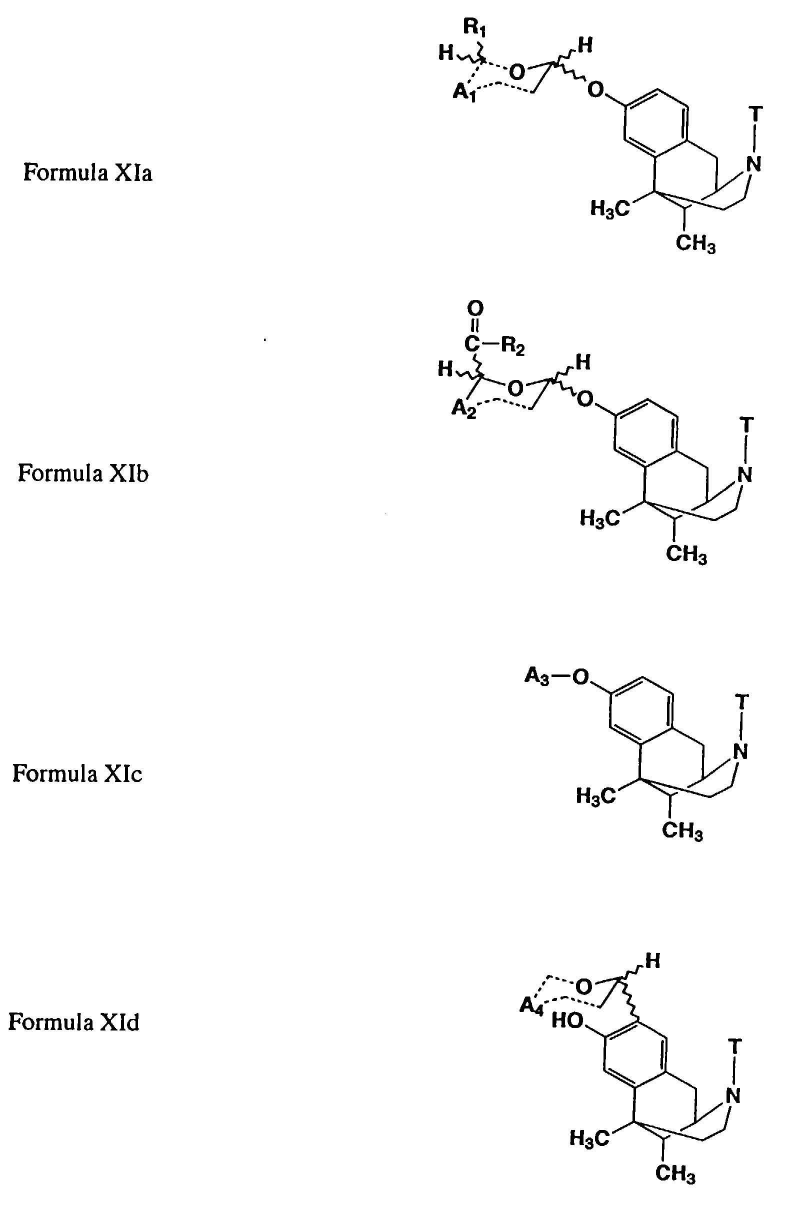

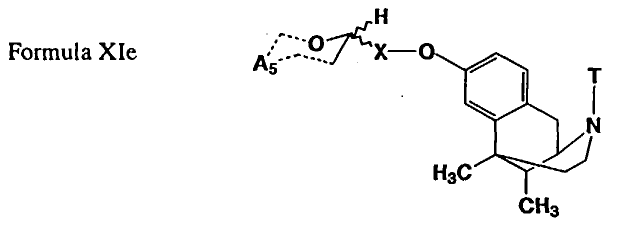

75. A compound of claim 74 which is pentazocyl-β-D-glucopyranoside acetate.

76. A compound of claim 74 which is pentazocyl-β-D-galactopyranoside acetate.

77. A compound of claim 74 which is pentazocyl-β-D-galactopyranosyl uronic acid acetate.

78. A compound of claim 74 which is pentazocyl-β-D-glucopyranosyl uronamide acetate.

79. A compound of claim 74 which is pentazocyl-β-D-galactopyranosyl uronamide acetate.

80. A compound of claim 74 which is cyclazocyl-β-D-glucopyranoside acetate.

81. A compound of claim 74 which is cyclazocyl-β-D-galactopyranoside acetate.

82. A compound of claim 74 which is cyclazocyl-β-D-galactopyranosyl uronic acid acetate.

83. A compound of claim 74 which is cyclazocyl-β-D-glucopyranosyl uronamide acetate.

84. A compound of claim 74 which is cyclazocyl-β-D-galactopyranosyl uronamide acetate.

85. A derivative according to anyone of claims 1 to 3 wherein the sugar residue is attached to an hydroxyl group of the opioid by a O-glycosidic bond.

86. A derivative according to anyone of claims 1 to 3 wherein the sugar residue is coupled to an hydroxyl group of the opiate by an ether function.

87. A derivative according to anyone of claims 1 to 3 wherein the sugar residue is linked to the opiate molecule by a C-glycosidic bond.

88. A derivative according to anyone of claims 1 to 20 wherein the opiate attached to the sugar residue posseses pharmacological and therapeutical activities in relation to analgesia, cough, dispnea, constipation, diarrhea and anesthesia or which stimulate or inhibit such activity.

89. A derivative according to claim 88 wherein the opiate has a stimulating or inhibitory pain/analgesic/antinociceptive activity.

90. A derivative according to claim 88 wherein the opiate has an inhibitory coughing activity.

91. A derivative according to claim 88 wherein the opiate has an inhibitory constipational activity.

92. A derivative according to claim 88 wherein the opiate has an inhibitory diarrheal activity.

93. A derivative according to claim 88 wherein the opiate has a stimulating or inhibitory activity in relation to dispnea.

94. A derivative according to claim 88 wherein the opiate has a stimulating anesthesic activity.

95. A process for the preparation of a sugar derivative of a biologically active opiate which has an enhaced analgesic potency if compared to the non-sugar modified opiate and carries at least one sugar residue which is attached by direct O-glycosidic bond or ether coupling to one hydroxyl group of the opiate or by means of a C-glycosidic bond between two other suitable functions on the two constituting moieties. Such a process is characterized by,
a) At least one protecting group on the molecule of the opiate glyconjugate is cleaved or,
b) A conveniently modified and/or protected sugar residue and a suitably derivatized opiate unit are linked together by glycosidic or ether or C-C bond, and stage a) of the process is optionally performed.
c) At least one optionally protected residue is introduced into a protected or unprotected opiate glycoconjugate and stage a) of the process is optionally effected, or
d) A functional group of a protected or unprotected opiate glycoconjugate is converted into another functional group or removed and a further residue is introduced, so that an unprotected or protected opiate glycoconjugate is obtained, and in the latter case, stage a) is followed.

96. A process for the production of a compound:
a) of Formula I 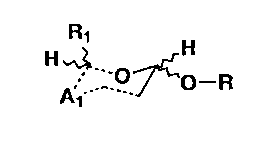 wherein 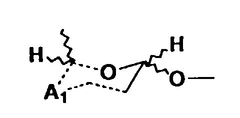 is a glycosyl residue being linked via glycosidic bond to an hydroxyl group of a biologically active opiate, R₁ is hydrogen, hydroxyl group, a saturated or insaturated or aromatic linear or branched or cyclic alcohol and/or acid residue with 1 to 18 C-atoms, a sulfate(s) or phosphate(s) residue(s) and R is the residue of a biologically active opiate of formula R-OH, wherein the OH group is either an alcohol or a phenol function.
b) of Formula II 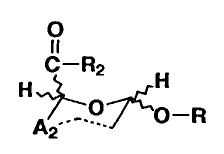 wherein 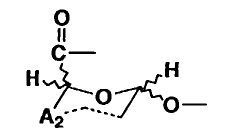 is a residue of an uronic acid, R₂ is hydroxyl, amino, a saturated or insaturated or aromatic linear or branched or cyclic amine or alcohol residue with 1 to 18 C-atoms and R is the residue of a biologically active opiate of formula R-OH, wherein the OH group is either an alcohol or a phenol function.
c) of Formula III
A₃-O-R
wherein A₃-O- is a glycosyl residue being linked via ether bond to an hydroxyl group of a biologically active opiate and R is the residue of a biologically active opiate of formula R-OH, wherein the OH group is either an alcohol or a phenol function.
d) of Formula IV 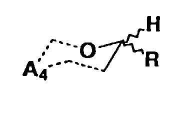 wherein 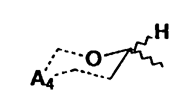 is a deoxy glycidyl residue being coupled via C-C single bond to an atom carbon of a biologically active opiate and R is the residue of a biologically active opiate.
e) of Formula V 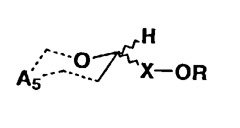 wherein 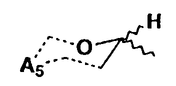 is a sugar residue, R denotes the residue of a biologically active opiate of formula R-OH and X is a linker group, i.e., NHCO or NHCS coupling the opiate to the sugar residue.
Acid salts and complexes of these opiate glycoconjugates of formula I to V are also among the compounds that can be prepared by this process. Such a process is characterized by,
a) At least one protecting group on the molecule of the opiate glyconjugate is cleaved or,
b) A conveniently modified and/or protected sugar residue and a suitably derivatized opiate unit are linked together by glycosidic or ether or C-C bond, and stage a) of the process is optionally performed.
c) At least one optionally protected residue is introduced into a protected or unproected opiate glycoconjugate and stage a) of the process is optionally effected, or
d) A functional group of a protected or unprotected opiate glycoconjugate is converted into another functional group or removed and a further residue is introduced, so that an unprotected or protected opiate glycoconjugate is obtained, and in the latter case, stage a) is followed.
The isolation of the compound is performed in free form, acid addition salt or complex form.

97. A process for the production of a sugar derivative of claim 1 essentially as here described and taken into account the methods outlined in any one of the examples.

98. A compound whenever produced by a process of anyone of claims 95 to 97.

99. A compound of any of claims 1 to 94 and 98 as a hydrate form

100. A compound of any of claims 1 to 94 and 98 in acid addition salt form.

101. A pharmaceutical composition containing a compound fo anyone of claims 1 to 94 and 98 in free form or in a biologically acceptable salt or complex form in association with a pharmaceutical carrier or galenic preparation.

102. A composition according to claim 101 adapted for oral administration.

103. A composition according to claim 101 adapted for nasal administration.

104. A compound according to anyone of claims 4 to 84 in a pharmaceutically acceptable form for use as a pharmaceutical preparation.
